(19)

(11) **EP 4 342 360 B1**

(12) **EUROPEAN PATENT SPECIFICATION**

(45) Date of publication and mention of the grant of the patent:
**31.12.2025 Bulletin 2026/01**

(21) Application number: **23198959.1**

(22) Date of filing: **22.09.2023**

(51) International Patent Classification (IPC):
***A61B 3/032*** *(2006.01)* ***A61B 3/15*** *(2006.01)*
***A61B 3/08*** *(2006.01)*

(52) Cooperative Patent Classification (CPC):
**A61B 3/152; A61B 3/032; A61B 3/085**

# (54) OPHTHALMIC APPARATUS

OPHTHALMISCHE VORRICHTUNG

APPAREIL OPHTALMIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **26.09.2022 JP 2022153119**
**16.06.2023 JP 2023099209**

(43) Date of publication of application:
**27.03.2024 Bulletin 2024/13**

(73) Proprietor: **TOPCON CORPORATION**
**Tokyo 174-8580 (JP)**

(72) Inventors:
- **YUKIMORI, Takafumi**
  **Tokyo, 174-8580 (JP)**
- **TATARA, Yoko**
  **Tokyo, 174-8580 (JP)**
- **SAIKA, Makoto**
  **Tokyo, 174-8580 (JP)**

(74) Representative: **Louis Pöhlau Lohrentz Patentanwälte**
**Merianstrasse 26**
**90409 Nürnberg (DE)**

(56) References cited:
**JP-A- 2016 158 721 JP-A- 2021 146 184**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 342 360 B1

## Description

### FIELD OF THE INVENTION

The present disclosure relates to an ophthalmic apparatus.

### BACKGROUND

Conventionally, it is known that strabismus or phoria is the cause of asthenopia. Furthermore, in some cases, a subject eye having strabismus or phoria fails to be fixed to an optotype, and ocular characteristics fail to be accurately acquired. Accordingly, an ophthalmic apparatus that suddenly switches shielding and transmitting visible light from/to subject eyes, forcibly switches binocular vision and monocular vision, measures adjustment of the subject eyes before and after switching by using invisible light, and measures a change in a line-of-sight direction in order to discover an abnormality in an eye position, such as strabismus or phoria, of the subject eyes has been disclosed (for example, see JP 5011144 B2). As described above, there is a request for the development of a technology that enables a state of a subject eye, such as an abnormality in an eye position, to be appropriately grasped.

JP 2016-158721 A discloses an ophthalmic apparatus with gaze direction detection using corneal bright spots and pupil centers when fixation targets are presented at different positions (paragraphs [0060]-[0061]).

JP 2021-146184 A discloses a binocular ophthalmic apparatus with pupil center and corneal reflection detection for line-of-sight determination (paragraphs [0080]-[0081]).

### SUMMARY

The present disclosure has been made in view of the circumstances described above, and it is an object of the present disclosure to provide an ophthalmic apparatus that is capable of appropriately grasping a state of a subject eye.

In order to achieve the object described above, an ophthalmic apparatus according to the present disclosure includes the features defined in claim 1.

An ophthalmic apparatus configured as described above enables a state of a subject eye to be appropriately grasped. As a result, a tester or the like can also appropriately grasp a condition of an eye position, such as strabismus or phoria, of the subject eyes.

### BRIEF DESCRIPTION OF DRAWINGS

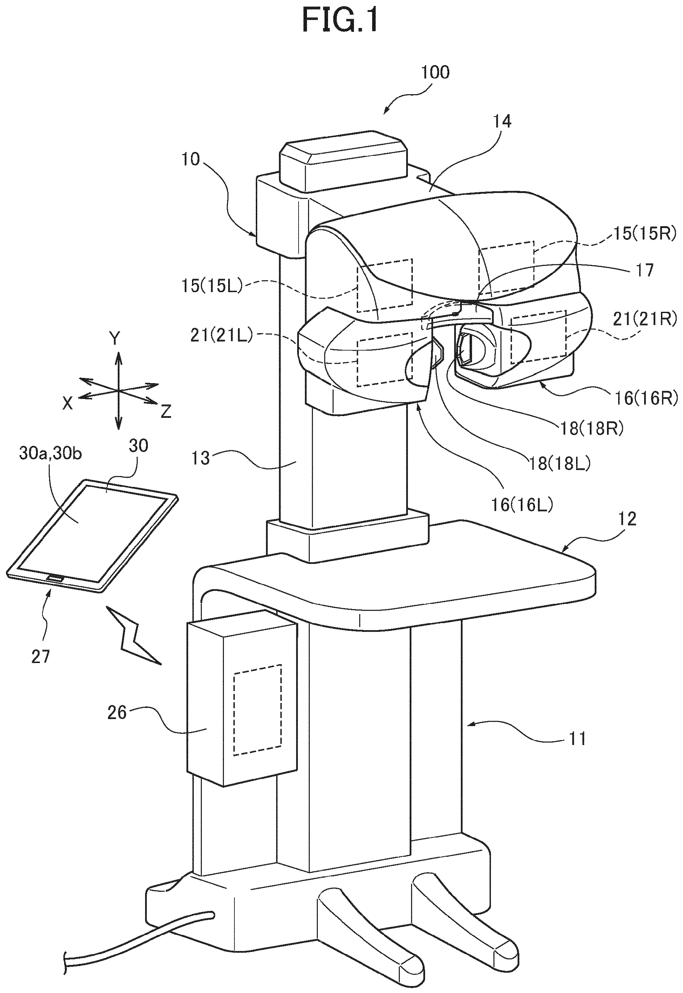

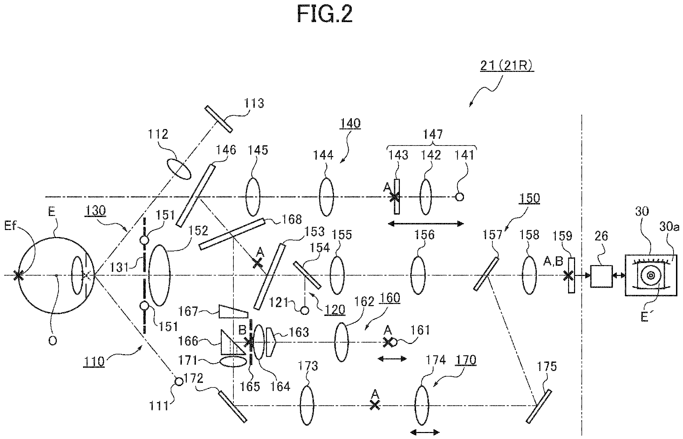

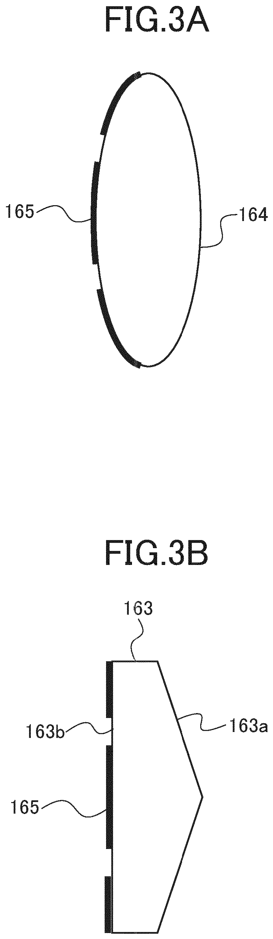

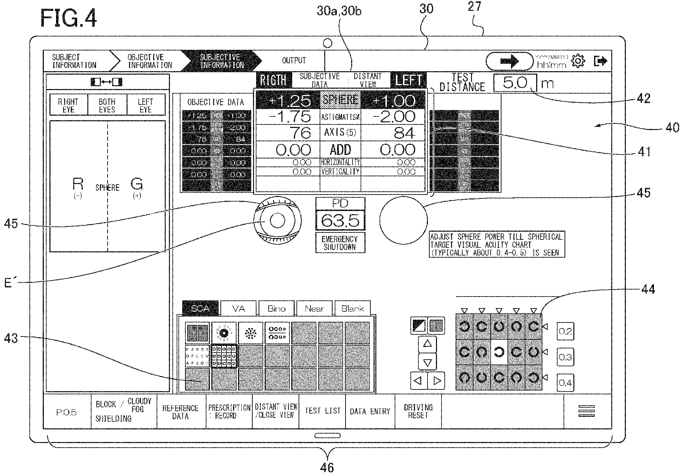

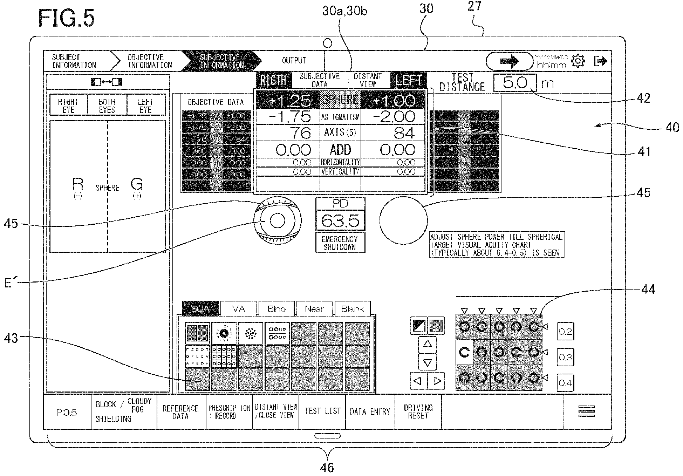

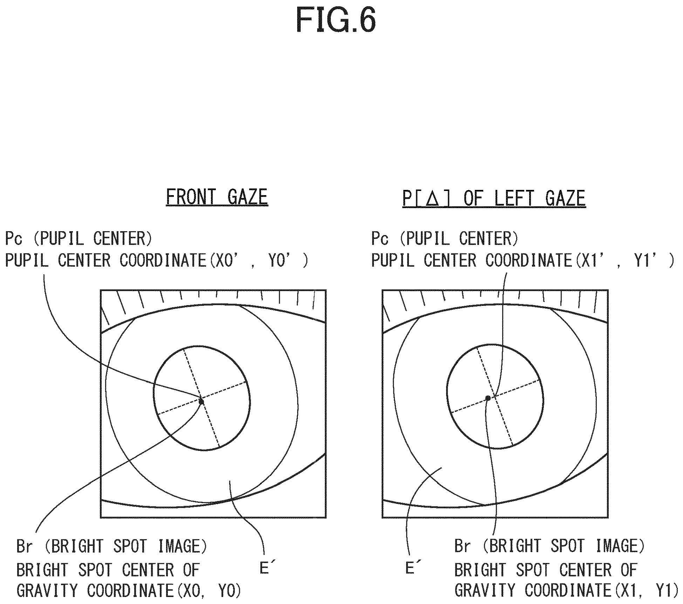

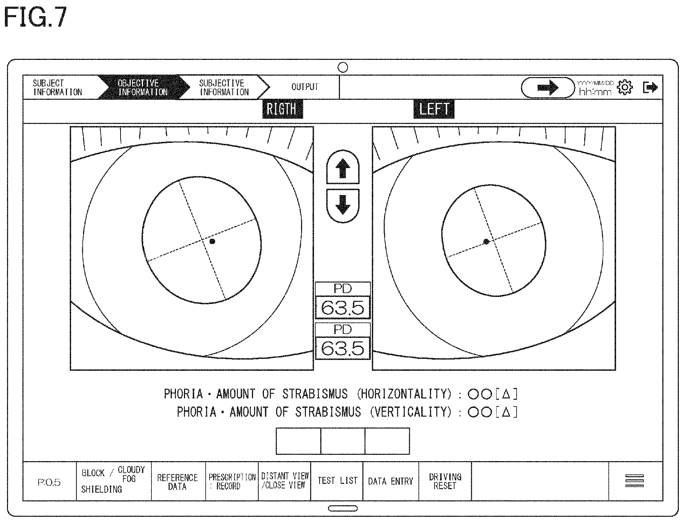

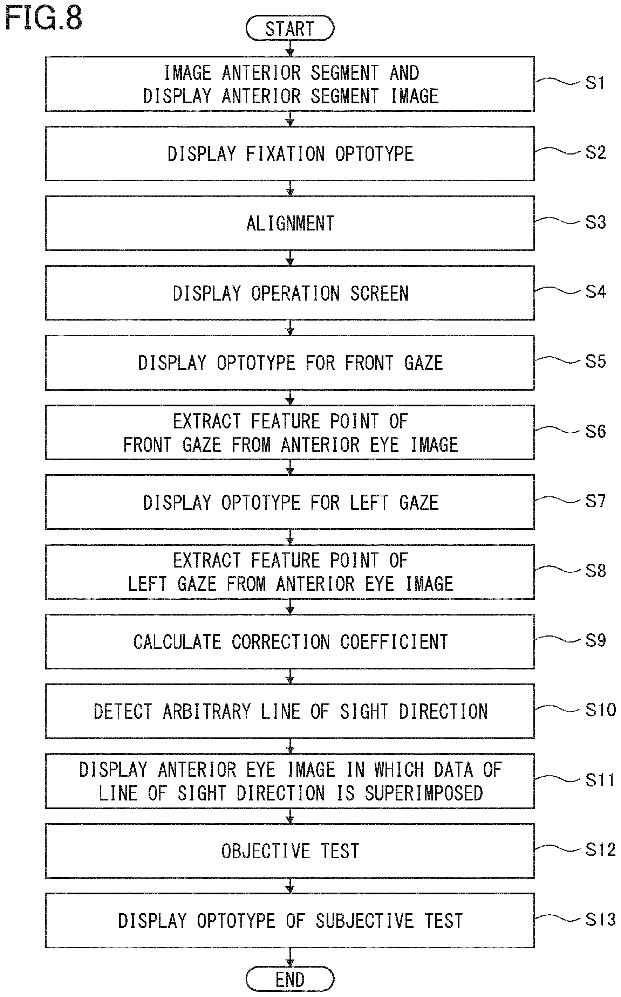

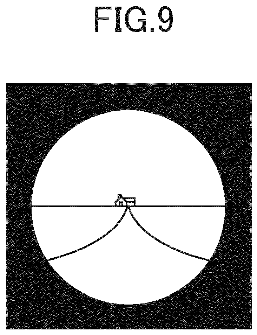

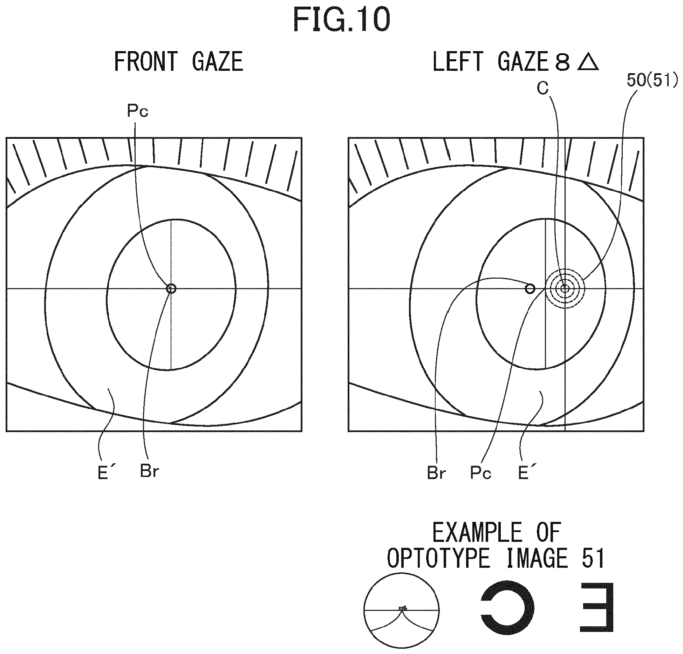

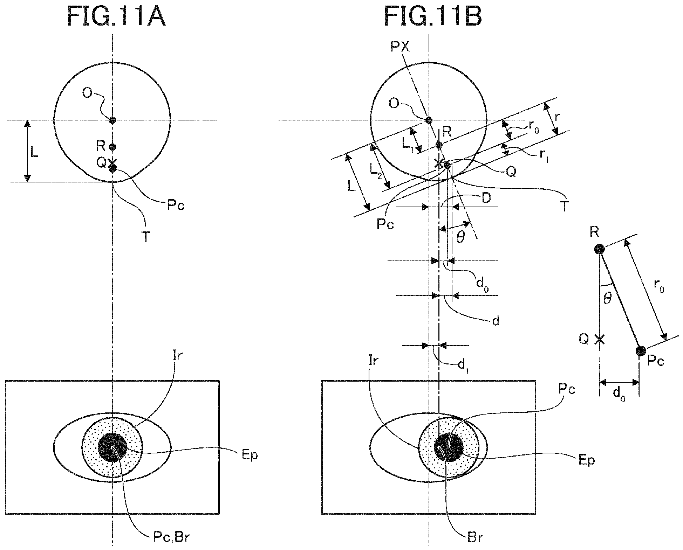

FIG. 1 is a perspective view illustrating the entire configuration of an ophthalmic apparatus according to a first embodiment. FIG. 2 is a diagram illustrating a detailed configuration of a right-eye measurement optical system of the ophthalmic apparatus according to the first embodiment. FIG. 3A is a diagram schematically illustrating a sectional view of the field lens of FIG. 2. FIG. 3B is a diagram schematically illustrating a sectional view of the conical prism of FIG. 2. FIG. 4 is a diagram illustrating an example of an operation screen that is displayed on a display screen of a display unit of the ophthalmic apparatus according to the first embodiment. FIG. 5 is a diagram illustrating an example of an operation screen that is displayed on the display screen of the display unit of the ophthalmic apparatus according to the first embodiment. FIG. 6 is an explanatory diagram for explaining a relationship between an anterior segment image of a subject eye in a front gaze and an anterior segment image of the subject eye in a left gaze, and bright-spot center-of-gravity coordinates and pupil center coordinates. FIG. 7 is a diagram illustrating an example of an information display screen that is displayed on the display screen of the display unit of the ophthalmic apparatus according to the first embodiment. FIG. 8 is a flowchart illustrating an example of an operation of the ophthalmic apparatus according to the first embodiment. FIG. 9 is an explanatory diagram for explaining another example of an optotype that is used in the ophthalmic apparatus according to the first embodiment. FIG. 10 is an explanatory diagram for explaining a relationship between an anterior segment image of a subject eye in a front gaze and an anterior segment image of the subject eye in a left gaze, and bright-spot center-of-gravity coordinates, pupil center coordinates, and a prism circle. FIGS. 11A and 11B are explanatory diagrams illustrating another method for detecting a line-of-sight direction.

### DETAILED DESCRIPTION

#### First Embodiment

An ophthalmic apparatus according to a first embodiment of the present disclosure is described below with reference to FIGS. 1 to 3B. An ophthalmic apparatus 100 according to the first embodiment is an ophthalmic apparatus of a binocular open type that can simultaneously perform the characteristics measurement of a subject eye E on both eyes in a state where a subject keeps both left and right eyes open. Note that the ophthalmic apparatus 100 according to the present embodiment can also conduct testing or the like on each single eye by shielding a single eye or turning off a fixation optotype. Furthermore, the ophthalmic apparatus is not limited to a binocular open type, and the present disclosure can be applied to an ophthalmic apparatus that performs characteristics measurement on each single eye.

The ophthalmic apparatus 100 according to the first embodiment is an apparatus that conducts arbitrary subjective testing, and can also conduct objective testing. Note that in the subjective testing, the ophthalmic apparatus 100 presents an optotype or the like to the subject in a predetermined presentation position, and acquires a testing result on the basis of a response of the subject to this optotype or the like. Examples of this subjective testing include subjective refraction measurement, such as long-distance testing, intermediate-distance testing, near-distance testing, contrast testing, night vision testing, glare testing, pinhole testing, or stereoscopic vision testing, visual field testing, and the like. Furthermore, in the objective testing, the ophthalmic apparatus 100 irradiates the subject eye E with light, and measures information (ocular characteristics) relating to the subject eye E on the basis of a result of detecting its feedback light. This objective testing includes measurement for acquiring characteristics of the subject eye E, and imaging for acquiring an image of the subject eye E (see FIG. 2). Moreover, examples of the objective testing include objective refraction measurement (refraction measurement), corneal shape measurement (keratometry), intraocular pressure measurement, ophthalmography, tomography using optical coherence tomography (hereinafter referred to as "OCT") (OCT imaging), measurement using OCT, and the like.

The entire configuration of the ophthalmic apparatus will be described. The ophthalmic apparatus 100 according to the present embodiment principally includes a body 10, a control unit 26 that is provided to the body 10, a tester controller 27, and a not-illustrated subject controller, as illustrated in FIG. 1. The body 10 includes a base 11, an optometry table 12, a pole 13, an arm 14, a pair of driving mechanisms (driving units) 15, a pair of measurement heads (measurement unit) 16, a forehead applied portion 17, and the control unit 26. The ophthalmic apparatus 100 acquires information relating to a subject eye E of a subject in a state where the subject facing the optometry table 12 is applying the forehead to the forehead applied portion 17 that is provided between both measurement heads 16. Note that herein, an X-axis, a Y-axis, and a Z-axis are set as illustrated in FIG. 1, and it is assumed that a leftward/rightward direction is an X-direction, an upward/downward direction (a vertical direction) is a Y-direction, and a direction that is orthogonal to the X-direction and the Y-direction (a depth direction of the measurement head 16) is a Z-direction, when viewed from the subject.

The optometry table 12 is a desk on which the tester controller 27 or the subject controller is placed or an object to be used in optometry is placed, and is supported by the base 11. The optometry table 12 may be supported by the base 11 in such a way that a position in the Y-direction (a height position) can be adjusted.

The pole 13 is supported by the base 11 to extend in the Y-direction at a rear end of the optometry table 12, and is provided with the arm 14 at a distal end. The arm 14 hangs both measurement heads 16 with the driving mechanisms 15 interposed therebetween above the optometry table 12, and extends from the pole 13 to a front side in the Z-direction. The arm 14 can move in the Y-direction relative to the pole 13. Note that the arm 14 may be able to move in the X-direction and the Z-direction relative to the pole 13. At a distal end of the arm 14, the pair of driving mechanisms 15 are hung. This pair of driving mechanisms 15 hang and support the pair of measurement heads 16.

The driving mechanisms 15 and the measurement heads 16 are provided in one-to-one correspondence to individually correspond to left-hand and right-hand subject eyes E of the subject. Hereinafter, the driving mechanisms 15 are individually referred to as a left-eye driving mechanism 15L and a right-eye driving mechanism 15R, and the measurement heads 16 are individually referred to as a left-eye measurement head 16L and a right-eye measurement head 16R. The left-eye driving mechanism 15L and the right-eye driving mechanism 15R, and the left-eye measurement head 16L and the right-eye measurement head 16R are plane-symmetric relative to a vertical plane that is located in an intermediate position of both driving mechanisms and both measurement heads in the X-direction.

The left-eye driving mechanism 15L hangs the left-eye measurement head 16L in a movable manner. The right-eye driving mechanism 15R hangs the right-eye measurement head 16R in a movable manner. The left-eye driving mechanism 15L and the right-eye driving mechanism 15R move the left-eye measurement head 16L and the right-eye measurement head 16R individually or in conjunction with each other in the Y-direction (the vertical direction) and in the X-direction and the Z-direction (a horizontal direction) on the basis of a control signal from the control unit 26. Furthermore, the left-eye driving mechanism 15L and the right-eye driving mechanism 15R rotate the left-eye measurement head 16L and the right-eye measurement head 16R individually or in conjunction with each other in the X-direction (the horizonal direction) by using, as a center (a rotation axis), a vertical eyeball rotation axis that passes through an eyeball rotation point O (see FIG. 2) of the subject eye E and extends in the vertical direction (the Y-direction), and in the Y-direction (the vertical direction and the upward/downward direction) by using, as a center (a rotation axis), a pair of left-hand and right-hand horizontal eyeball rotation axes that pass through the eyeball rotation point O of the subject eye E and extend in the horizontal direction (the X-direction), on the basis of a control signal from the control unit 26.

As described above, the pair of driving mechanisms 15 rotate the pair of measurement heads 16 in the X-direction to enable the subject eyes E to diverge (a divergence movement) or converge (a convergence movement). Furthermore, the pair of driving mechanisms 15 rotate the pair of measurement heads 16 in the Y-direction to enable lines of sight of the subject eyes E to face a downward direction or return to original positions. By doing this, the ophthalmic apparatus 100 conducts divergence movement and convergence movement tests on the subject, or conducts testing using various testing distances that range from long-distance testing using a long distance to near-distance testing using a near distance in a binocular vision state on the subject to be able to measure various characteristics of both subject eyes E.

The left-eye measurement head 16L acquires information relating to a left-hand side subject eye E of the subject, and the right-eye measurement head 16R acquires information relating to a right-hand side subject eye E of the subject.

The respective measurement heads 16 include measurement optical systems 21 (individually referred to as a right-eye measurement optical system 21R and a left-eye measurement optical system 21L) that acquire eye information of the subject eye E. Each of the measurement heads 16 includes a mirror 18 (18L or 18R) serving as a deflection member, and the measurement optical system 21 acquires information relating to a corresponding subject eye E by using the mirror 18.

Each of the measurement optical systems 21 (the left-eye measurement optical system 21L and the right-eye measurement optical system 21R) is constituted by one or a combination of some of a visual acuity testing device that conducts visual acuity testing while switching optotypes to be presented, a phoropter that acquires appropriate corrective refractive power of a subject eye E while switching and disposing a corrective lens, a refractometer or a wavefront sensor that measures refractive power, a fundus camera that captures an image of the fundus of the eye, a tomographic device that captures a tomographic image of the retina, a specular microscope that captures a corneal endothelium image, a keratometer that measures a corneal shape, a tonometer that measures intraocular pressure, and the like.

Detailed configurations of the left-eye measurement optical system 21L and the right-eye measurement optical system 21R are described below with reference to FIG. 2. In FIG. 2, the mirror 18R is omitted. Note that the detailed configurations of the left-eye measurement optical system 21L and the right-eye measurement optical system 21R are not limited to the configuration illustrated in FIG. 2. Furthermore, the left-eye measurement optical system 21L and the right-eye measurement optical system 21R have the same configuration. Therefore, a description of the left-eye measurement optical system 21L is omitted below, and only the right-eye measurement optical system 21R is described.

Furthermore, in the description below, it is assumed that a "fundus conjugated position A" is a position that is roughly optically conjugated with the fundus Ef of the subject eye E in a state where alignment has been completed, and means a position that is optically conjugated with the fundus Ef of the subject eye E and its proximity. It is assumed that a "pupil conjugated position B" is a position that is roughly optically conjugated with the pupil of the subject eye E in a state where alignment has been completed, and means a position that is optically conjugated with the pupil of the subject eye E and its proximity.

The right-eye measurement optical system 21R includes a Z-alignment system 110, an XY-alignment system 120, a keratometry system 130, an optotype projection system 140, an anterior segment observation system 150, a refraction measurement projection system 160, and a refraction measurement light-receiving system 170, as illustrated in FIG. 2.

The anterior segment observation system 150 will be described. The anterior segment observation system 150 captures a moving image of an anterior segment of the subject eye E. In an optical system that passes through the anterior segment observation system 150, an imaging plane of an imaging element 159 serving as an image acquisition unit is disposed in the pupil conjugated position B. An anterior segment illumination light source 151 irradiates the anterior segment of the subject eye E with illumination light (for example, infrared light) that is constituted by a parallel light flux. Light that has been reflected by the anterior segment of the subject eye E passes through an objective 152, is transmitted through a first dichroic mirror 153, is transmitted through a half mirror 154, passes through a first relay lens 155 and a second relay lens 156 in order, and is transmitted through a second dichroic mirror 157. Light that has been transmitted through the second dichroic mirror 157 is formed as an image on the imaging plane of the imaging element 159 (an area sensor) by a first tube lens 158. The imaging element 159 performs imaging and signal outputting at a predetermined rate. An output (a video signal) of the imaging element 159 is input to the control unit 26. The control unit 26 causes a display screen 30a of a display unit 30 to display an anterior segment image E' based on this video signal. The anterior segment image E' is, for example, an infrared moving image.

The Z-alignment system 110 will be described. The Z-alignment system 110 projects light (infrared light) for alignment in an optical axis direction (the forward/backward direction and the Z-direction) of the anterior segment observation system 150 onto the subject eye E. Light that has been output from a Z-alignment light source 111 is projected onto the cornea of the subject eye E, is reflected by the cornea, and is formed as an image on a sensor face of a line sensor 113 by a second tube lens 112. If a position of a corneal apex has changed in the optical axis direction of the anterior segment observation system 150, a position of projection of light on the sensor face of the line sensor 113 changes. The control unit 26 obtains a position of the corneal apex of the subject eye E on the basis of the position of projection of light on the sensor face of the line sensor 113, and controls the driving mechanism 15 that moves the measurement optical system 21 on the basis of this to perform Z-alignment.

The XY-alignment system 120 will be described. The XY-alignment system 120 irradiates the subject eye E with light (infrared light) for alignment in a direction that is orthogonal to the optical axis of the anterior segment observation system 150 (the leftward/rightward direction (the X-direction) and the upward/downward direction (the Y-direction)). The XY-alignment system 120 includes an XY-alignment light source 121 that is provided on a light path that has been branched from the anterior segment observation system 150 by the half mirror 154. Light that has been output from the XY-alignment light source 121 is reflected by the half mirror 154, and is projected onto the subject eye E through the anterior segment observation system 150. Light that has been reflected by the cornea of the subject eye E is guided to the imaging element 159 through the anterior segment observation system 150.

An image (a bright spot image) based on this reflected light is included in the anterior segment image E'. The control unit 26 causes the display screen 30a of the display unit 30 to display the anterior segment image E' including the bright spot image and an alignment mark. In a case where XY-alignment is manually performed, the tester or the like performs an operation to move the measurement optical system in such a way that the bright spot image is guided into the alignment mark. In the case of automated alignment, the control unit 26 controls the driving mechanism 15 that moves the measurement optical system 21 in such a way that the displacement of the bright spot image relative to the alignment mark is canceled.

The Keratometry system 130 will be described. The keratometry system 130 projects a ring-shaped light flux (infrared light) for measuring a shape of the cornea of the subject eye E onto the cornea. A keratometry plate 131 is disposed between the objective 152 and the subject eye E. On a back face side (a side of the objective 152) of the keratometry plate 131, a keratometry ring light source (not illustrated) is provided. Light from the keratometry ring light source illuminates the keratometry plate 131, and this causes the ring-shaped light flux to be projected onto the cornea of the subject eye E. Reflected light (a keratometry ring image) from the cornea of the subject eye E is detected together with the anterior segment image E' by the imaging element 159. The control unit 26 performs a publicly known arithmetic operation on the basis of this keratometry ring image to calculate a corneal shape parameter indicating the shape of the cornea.

The optotype projection system 140 will be described. The optotype projection system 140 presents a fixation optotype to the subject eye E. Light (visible light) that has been output from a light source 141 is changed to a parallel light flux by a collimator lens 142, and is applied to an optotype chart 143. The optotype chart 143 includes, for example, a transmission type liquid crystal panel, and displays a pattern indicating the optotype. Light that has been transmitted through the optotype chart 143 passes through a third relay lens 144 and a fourth relay lens 145 in order, is reflected by a first reflection mirror 146, is transmitted through a third dichroic mirror 168, and is reflected by the first dichroic mirror 153. Light that has been reflected by the first dichroic mirror 153 passes through the objective 152, and is projected onto the fundus Ef. The light source 141, the collimator lens 142, and the optotype chart 143 constitute an optotype unit 147, and can integrally move in the optical axis direction.

In the case of subjective testing, the control unit 26 moves the optotype unit 147 in the optical axis direction on the basis of a result of objective measurement, and controls the optotype chart 143. The control unit 26 causes the optotype chart 143 to display an optotype selected by a tester or the control unit 26. As a result, the optotype is presented to the subject. The subject responds to the optotype. Upon receipt of an input of the content of response, the control unit 26 performs further control, or calculates a subjective testing value. For example, in visual acuity measurement, the control unit 26 selects and presents the next optotype on the basis of a response to a Landolt ring or the like, and repeats this selection and presentation to determine a visual acuity value.

Furthermore, the optotype displayed by the optotype chart 143 is not particularly limited if the optotype is used for optometry, and suitable examples include a Landolt ring, a Snellen chart, an E chart, and the like. As the optotype, a variety of optotypes including a letter such as hiragana or katakana, an optotype constituted by a picture or the like indicating an animal, a finger, or the like, a specified figure for binocular vision function testing such as a cross optotype, an optotype constituted by, for example, a landscape painting or a landscape photograph, or the like, can be used. Furthermore, the optotype may be a still image or may be a moving image. In the present embodiment, the optotype chart 143 includes the liquid crystal panel. Therefore, an optotype having a desired shape, form, or contrast can be displayed at a predetermined testing distance, and diversified minute optometry can be performed. Furthermore, the ophthalmic apparatus 100 includes two optotype units 147 (the optotype charts 143) that correspond to left-hand and right-hand subject eyes E, and therefore an optotype that generates parallax can be displayed to correspond to a predetermined testing distance (a presentation position of the optotype), and stereoscopic vision testing can also be easily and precisely conducted in a natural orientation of an optic axis.

The refraction measurement projection system 160 and the refraction measurement light-receiving system 170 will be described. The refraction measurement projection system 160 and the refraction measurement light-receiving system 170 are used for objective refraction measurement (refraction measurement). The refraction measurement projection system 160 projects a ring-shaped light flux (infrared light) for objective measurement onto the fundus Ef. The refraction measurement light-receiving system 170 receives feedback light from the subject eye E of this ring-shaped light flux.

A refraction measurement light source 161 may be a superluminescent diode (SLD) light source, which is a high-luminance light source in which a light-emitting diameter has a predetermined size or less. The refraction measurement light source 161 can move in the optical axis direction, and is disposed in the fundus conjugated position A. A ring diaphragm 165 (specifically, a light-transmitting portion) is disposed in the pupil conjugated position B. A focusing lens 174 can move in the optical axis direction. The focusing lens 174 may be a publicly known varifocal lens that is controlled by the control unit 26 to be able to change a focal position. In an optical system that passes through the refraction measurement light-receiving system 170, the imaging plane of the imaging element 159 is disposed in the fundus conjugated position A.

Light that has been output from the refraction measurement light source 161 passes through a fifth relay lens 162, and enters a conical face of a conical prism 163. Light that has entered the conical face is deflected, and is emitted from a bottom face of the conical prism 163. Light that has been emitted from the bottom face of the conical prism 163 passes through a field lens 164, and passes through the light-transmitting portion that has been formed in a ring shape in the ring diaphragm 165. Light (a ring-shaped light flux) that has passed through the light-transmitting portion of the ring diaphragm 165 is reflected by a reflection face of an apertured prism 166, passes through a rotary prism 167, and is reflected by the third dichroic mirror 168. Light that has been reflected by the third dichroic mirror 168 is reflected by the first dichroic mirror 153, passes through the objective 152, and is projected onto the subject eye E. The rotary prism 167 is used to average an amount-of-light distribution of the ring-shaped light flux for a blood vessel or a disease portion of the fundus Ef, or reduce speckle noise caused by a light source.

It is desirable that the conical prism 163 be disposed in a position that is as close as possible to the pupil conjugated position B.

In the field lens 164, for example, as illustrated in FIG. 3A, the ring diaphragm 165 may be stuck on a lens face on a side of the subject eye E. In this case, for example, in the field lens 164, a light shielding film is vapor-deposited on the lens face to form the ring-shaped light-transmitting portion.

Furthermore, the refraction measurement projection system 160 may have a configuration in which the field lens 164 is omitted.

Moreover, in the conical prism 163, for example, as illustrated in FIG. 3B, the ring diaphragm 165 may be stuck on a bottom face 163b of the conical prism 163 in which light that has passed through the fifth relay lens 162 enters a conical face 163a. In this case, for example, in the conical prism 163, a light shielding film is vapor-deposited on the bottom face 163b to form the ring-shaped light-transmitting portion. Furthermore, the ring diaphragm 165 may be disposed on a side of the conical face 163a of the conical prism 163.

The ring diaphragm 165 may be a diaphragm that includes a light-transmitting portion having a shape that corresponds to a predetermined measurement pattern. The ring diaphragm 165 may include the light-transmitting portion in a position that is eccentric relative to an optical axis of the refraction measurement projection system 160. Furthermore, the ring diaphragm 165 may include two or more light-transmitting portions.

Feedback light of the ring-shaped light flux that has been projected onto the fundus Ef passes through the objective 152, and is reflected by the first dichroic mirror 153 and the third dichroic mirror 168. Feedback light that has been reflected by the third dichroic mirror 168 passes through the rotary prism 167, passes through an aperture of the apertured prism 166, and passes through a sixth relay lens 171. Feedback light that has passed through the sixth relay lens 171 is reflected by a second reflection mirror 172, and passes through a seventh relay lens 173 and the focusing lens 174. Light that has passed through the focusing lens 174 is reflected by a third reflection mirror 175, is reflected by the second dichroic mirror 157, and is formed as an image on the imaging plane of the imaging element 159 by the first tube lens 158. The control unit 26 performs a publicly known arithmetic operation on the basis of an output from the imaging element 159 to calculate a refractive power value of the subject eye E. For example, the refractive power value includes sphere power, cylindrical power, and the angle of an astigmatic axis.

Between the apertured prism 166 and the sixth relay lens 171, a diaphragm (not illustrated) that restricts the diameter of a light flux on the pupil is disposed. A light-transmitting portion of this diaphragm is disposed in the pupil conjugated position B.

The control unit 26 moves each of the refraction measurement light source 161 and the focusing lens 174 in the optical axis direction on the basis of the calculated refractive power value in such a way that the fundus Ef, the refraction measurement light source 161, and the imaging plane of the imaging element 159 are optically conjugated with each other. Moreover, the control unit 26 moves the optotype unit 147 in its optical axis direction in accordance with a movement of the refraction measurement light source 161 and the focusing lens 174. The light source 141, the optotype unit 147 including the collimator lens 142 and the optotype chart 143, the refraction measurement light source 161, and the focusing lens 174 may be able to move in their optical axis directions in conjunction with each other.

The tester controller 27 is equipment that the tester serving as an operator uses to operate the ophthalmic apparatus 100. The tester controller 27 is an information processing apparatus that includes a computer including a CPU, a storage device, and the like. The tester controller 27 according to the first embodiment is constituted by a tablet terminal. Note that the tester controller 27 is not limited to the tablet terminal, and can be a smartphone or another portable information terminal, or can be a laptop personal computer, a desktop personal computer, or the like. Furthermore, the tester controller 27 can be a controller dedicated to the ophthalmic apparatus 100.

In the ophthalmic apparatus 100 according to the present embodiment, the tester controller 27 is configured to be portable. The tester may operate the tester controller 27 in a state disposed on the optometry table 12, or may hold and operate the tester controller 27 in the hand.

The tester controller 27 includes the display unit (a display panel) 30 that is constituted by a touch panel display. This display unit 30 includes the display screen 30a on which an image or the like is displayed, and an input unit 30b of a touch panel type that is disposed to be superimposed onto this display screen 30a. The display unit 30 itself is a single input unit, and the display screen 30a of the display unit 30 functions as the input unit 30b that receives an input operation including a touch operation performed by the tester. The input unit 30b also functions as a sensing face that detects a touch operation performed by the tester by using a finger, a stylus, or the like.

The tester controller 27 can perform short-range communication with the control unit 26 by using a communication means such as short-range wireless communication. The tester controller 27 displays, on the display screen 30a, a predetermined screen (for example, the operation screen 40 illustrated in FIGS. 4 and 5, the information display screen 40A illustrated in FIG. 7, or the like), or various images such as the anterior segment image E' acquired by the imaging element 159 of the measurement optical system 21 on the basis of a display control signal transmitted from the control unit 26. Furthermore, the tester controller 27 receives an operation input performed on the display screen 30a (the input unit 30b) by the tester, and transmits, to the control unit 26, input information (a control signal) that corresponds to this operation input.

FIGS. 4 and 5 are diagrams illustrating an example of the operation screen 40 displayed on the display screen 30a. The operation screen 40 includes a correction value setting region 41 in which a correction value, such as sphere power (S), cylinder power (C), an astigmatic axis (A), or addition (ADD), of the subject eye E is set, a testing distance setting region 42 in which a testing distance is set, optotype icons 43 for selecting optotypes, an optotype display region 44 in which a selected optotype is displayed, an anterior segment image display region (an optometry window) 45 in which an anterior segment image E' captured by the imaging element 159 is displayed, various operation buttons 46, or the like.

FIG. 7 is a diagram illustrating an example of the information display screen 40A displayed on the display screen 30a. This information display screen 40A includes anterior segment image display regions 47 in which left-hand and right-hand anterior segment images E' are displayed, and a line-of-sight direction display region 48 in which a result (an amount of phoria or an amount of strabismus) of detecting a line-of-sight direction is displayed.

The subject controller is equipment that a subject uses to make a response in acquiring various types of eye information of the subject eye E. The subject controller includes, for example, a keyboard, a mouse, a joystick, a touch pad, a touch panel, or the like that is not illustrated. The subject controller is connected to the control unit 26 via a wired or wireless channel, and transmits, to the control unit 26, input information (a control signal) that corresponds to an operation performed on the subject controller.

The control unit 26 is an information processing apparatus that is provided below the optometry table 12. The control unit 26 comprehensively controls each unit of the ophthalmic apparatus 100 including the measurement head 16 and the driving mechanism 15. Furthermore, the control unit 26 controls the driving mechanism 15 and the measurement head 16 on the basis of a control signal transmitted from the tester controller 27, causes the measurement head 16 to measure ocular characteristics of the subject eye E, and transmits a measurement result to the tester controller 27.

Furthermore, the control unit 26 controls each of the optotype projection systems 140 of the left-eye measurement optical system 21L and the right-eye measurement optical system 21R, causes an optotype to be presented to the subject eye E in at least two different presentation positions on an XY-plane, and changes a line-of-sight direction of the subject eye E. Furthermore, when an optotype has been presented in each of the presentation positions, the control unit 26 controls the anterior segment observation system 150 to cause the imaging element 159 to acquire the anterior segment image E' of the subject eye E. The control unit 26 extracts a feature point from each of the anterior segment images E' acquired by the imaging element 159, detects positional information on the anterior segment image E' of the extracted feature point, and detects a line-of-sight direction of the subject eye E based in the positional information. Stated another way, the control unit 26 functions as a line-of-sight direction detection unit. The control unit 26 causes the display screen 30a of the display unit 30 to display information relating to the detected line-of-sight direction together with the anterior segment images E', and present the information and the anterior segment images E' to the tester or the like.

Furthermore, the control unit 26 detects information relating to an eye position of the subject eyes on the basis of the detected line-of-sight direction. An example of the information relating to the eye position include an amount of phoria or an amount of strabismus (a prism amount). The control unit 26 can also cause the display screen 30a of the display unit 30 to display this information relating to the eye position, and the tester or the like can appropriately grasp the subject eyes E having phoria or strabismus, or a level (a condition) of phoria or strabismus.

Furthermore, an example of the feature point is a feature point (a first feature point) acquired from a bright spot image (also referred to as a Purkinje image), which is corneal reflex based on a point image acquired by forming, as an image, a parallel light flux that has entered the subject eye E (a light flux that is parallel to an optical axis that enters the subject eye E from the anterior segment observation system 150 of the measurement optical system 21) in the subject eye E. Another example of the feature point is a feature point (a second feature point) acquired from the pupil (a pupil image) detected from the anterior segment image E'. The control unit 26 detects the line-of-sight direction of the subject eyes E on the basis of the first feature point and the second feature point.

For example, the first feature point is positional information (bright-spot center-of-gravity coordinates) relating to the center of gravity of corneal reflex (the bright spot image), and the second feature point is positional information (pupil center coordinates) relating to a center of the pupil. The bright-spot center-of-gravity coordinates and the pupil center coordinates can be calculated on the basis of the anterior segment image E' by using a publicly known technique. The control unit 26 obtains a difference between these pieces of positional information, and calculates the line-of-sight direction of the subject eye E on the basis of this difference.

Specifically, for example, the control unit 26 detects the bright-spot center-of-gravity coordinates (X, Y) as the first feature point, and detects the pupil center coordinates (X', Y') as the second feature point. Then, the control unit 26 calculates a prism amount (unit: Δ (prism diopter)) in the horizontal direction and a prism amount [Δ] in the vertical direction of a line-of-sight direction of a subject eye relative to a predetermined reference direction according to Formulae (1) and (2) described below. In Formulae (1) and (2) described below, a, b, a', and b' are correction coefficients, and a = a' and b = b' may be established.

$$\text{Line-of-sight direction (in horizontal direction) of subject eye } [\Delta] = a*(X' - X) + b \qquad (1)$$

$$\text{Line-of-sight direction (in vertical direction) of subject eye } [\Delta] = a'*(Y' - Y) + b' \qquad (2)$$

The correction coefficients a and b are correction coefficients that are used to detect a line-of-sight direction in the horizontal direction, and are calculated according to, for example, Formulae (3) and (4) described below. In Formulae (3) and (4) described below, X0 and Y0 are an X-coordinate and a Y-coordinate of the center of gravity of a bright spot of a bright spot image Br, for example, in a front gaze (a line-of-sight direction at a time when an optotype is presented in a first presentation position), and X0' and Y0' are an X-coordinate and a Y-coordinate of a pupil center Pc in the front gaze. Furthermore, X1 and Y1 are an X-coordinate and a Y-coordinate of the center of gravity of a bright spot, for example, in a left gaze (a line-of-sight direction at a time when an optotype is presented in a second presentation position), and X1' and Y1' are an X-coordinate and a Y-coordinate of the pupil center Pc in the left gaze. P is a known prism amount [Δ], and is specifically a prism amount of an optotype that is presented to the subject eye E.

$$a = P/((X1' - X1) - (X'0 - X0)) \qquad (3)$$

$$b = -P*(X0' - X0)/((X1' - X1) - (X'0 - X0)) \qquad (4)$$

Here, a relationship between an anterior segment image E' of the subject eye E in the front gaze and an anterior segment image E' of the subject eye E in the left gaze, and the bright-spot center-of-gravity coordinates and the pupil center coordinates is described below with reference to FIG. 6. In FIG. 6, Br is a bright spot image, and Pc is a pupil center. The "front gaze" refers to a state where the subject eye E faces the front (a direction that is parallel to an optical axis of the measurement optical system 21). The "left gaze" refers to a state where the subject eye E faces a direction that crosses the optical axis, and faces a leftward direction. Similarly, a "right gaze", an "up gaze", and a "down gaze" respectively refer to states where the subject eye E faces directions that cross the optical axis, and faces a rightward direction, an upward direction, and a downward direction.

Examples of a display of an optotype for setting the line-of-sight direction of the subject eye E to the "front gaze", the "left gaze", the "right gaze", the "up gaze", and the "down gaze" are described below with reference to FIGS. 4 and 5. The operation screen 40 illustrated in FIGS. 4 and 5 indicates a state where a visual acuity chart in which optotypes are indicated in three rows and five columns (what is called "character arrangement optotypes") has been selected from the optotype icons 43, and has been displayed in the optotype display region 44. The tester selects an optotype to be presented to the subject eye E from the visual acuity chart having three rows and five columns, by performing a tap operation, a button operation, or the like. In the character arrangement optotypes having three rows and five columns, prism amounts of individual optotypes have been predetermined. An optotype in the center (in the first row and the third column) is an optotype having a prism amount of 0Δ in the horizontal direction and the vertical direction, and a difference in the prism amount between optotypes that are adjacent to each other on the upper and lower sides or the left-hand and right-hand sides is 1.28Δ. Stated another way, from the optotype in the center, a prism amount in the horizontal direction of each of the optotypes increases by 1.28Δ in each shift in the horizontal direction (the leftward/rightward direction), and a prism amount in the vertical direction of each of the optotypes increases by 1.28Δ in each shift in the vertical direction (the upward/downward direction). Note that a difference in the prism amount between adjacent optotypes is not limited to 1.28Δ, and an appropriate prism amount, such as 2Δ, 4Δ, or 8Δ, can be employed according to the purpose of detection of the line-of-sight direction, the purpose of testing, or the like.

When the line-of-sight direction of the subject eye E is set to the "front gaze", the tester selects an optotype (a prism mount of 0Δ) in the center of the optotype display region 44. In response to this selection, as illustrated in FIG. 4, the control unit 26 brightly displays the optotype in the center of the optotype display region 44, darkly displays the other optotypes, and controls the optotype projection system 140 to cause the optotype to be displayed in a presentation position in the center of the optotype chart 143 (the first presentation position). The subject eye E is fixed to this optotype displayed in the optotype chart 143, and this enables the line-of-sight direction to be set to the "front gaze".

On the other hand, when the line-of-sight direction of the subject eye E is set to the "left gaze", the tester selects, for example, an optotype in the second row and the first column of the character arrangement optotypes. This optotype has a prism amount in the horizontal direction of 2.56Δ (stated another way, in the Formulae (3) and (4), P = 2.56Δ is established). In response to this selection, as illustrated in FIG. 5, the control unit 26 brightly displays the optotype in the second row and the first column of the optotype display region 44, darkly displays the other optotypes, and controls the optotype projection system 140 to cause the optotype to be displayed in a presentation position on a left-hand side in the optotype chart 143 (the second presentation position). The subject eye E is fixed to this optotype displayed in the optotype chart 143, and this enables the line-of-sight direction to be set to the "left gaze".

Note that in the description above, the control unit 26 changes a presentation position of an optotype on the optotype chart 143 to change the line-of-sight direction of the subject eye E. However, the control unit 26 may present all of the optotypes on the optotype chart 143, and the tester may instruct that the subject fix the eye to an optotype in the center, and may instruct that the subject fix the eye to the optotype in the second row and the first column.

The correction coefficients a and b that are used for the line-of-sight direction in the horizontal direction (the leftward/rightward direction) can also be calculated on the basis of each of the anterior segment images E' of the "front gaze" and the "right gaze". Furthermore, the correction coefficients a' and b' that are used for the line-of-sight direction in the vertical direction (the upward/downward direction) can be calculated on the basis of each of the anterior segment images E' of the "front gaze" and the "up gaze" or each of the anterior segment images E' of the "front gaze" and the "down gaze". Furthermore, the correction coefficients can be calculated from a calculation result based on each of the anterior segment images E' in three or more positions (for example, the "front gaze", the "right gaze", and the "left gaze") according to a least-squares method. Furthermore, the correction coefficients a and b and the correction coefficients a' and b' can also be simultaneously calculated by using an optotype in the center and an optotype that causes a line of sight to face an oblique direction (for example, an optotype in the first row and the second column, an optotype in the third row and the second column, an optotype in the first row and the fourth column, and/or an optotype in the third row and the fourth column) that are illustrated in FIG. 5 or the like.

A procedure of detecting the line-of-sight direction, as described above, can be used in any cases of a subject's face (head), and the procedure can be suitably used, in particular, when the subject's face is not fixed. An example of a state where "the subject's face is not fixed" is a state where an orientation or the like of the face is not appropriate, such as a state where the face is unsteady, a state where the face faces a lateral direction, or a state where the head is inclined in a leftward or rightward direction, regardless of whether the forehead abuts onto the forehead applied portion 17. In such a state, similarly, the control unit 26 can appropriately calculate the line-of-sight direction by using a plurality of feature points based on a bright spot, the pupil, and the like that have been extracted from the anterior segment image E'. Note that in a case where the subject's face is fixed to an appropriate state (for example, a state where the face does not unexpectedly move, and faces the front, and the head is not inclined, and is held erect) by the forehead applied portion 17, a chin receiving portion, or the like, the control unit 26 can appropriately calculate the line-of-sight direction even in the case of a single feature point (for example, a feature point based on a pupil image). Moreover, the used of a plurality of feature points enables the control unit 26 to more appropriately calculate the line-of-sight direction.

An example of an operation performed in the ophthalmic apparatus 100 according to the first embodiment that has the configuration described above is described below with reference to the flowchart of FIG. 8. Note that it is assumed that a power source is turned on to activate the ophthalmic apparatus 100, and the control unit 26 is communicable with the tester controller 27 and the subject controller.

In testing, the tester allows a subject to sit on a chair or the like, face the ophthalmic apparatus 100, and apply the forehead to the forehead applied portion 17. The operation illustrated in the flowchart of FIG. 8 is started, for example, at a timing at which a sensor or the like has sensed that the subject has applied the forehead to the forehead applied portion 17 or at a timing at which the tester has issued an imaging instruction by using the operation screen.

First, in step S1, the control unit 26 controls the anterior segment observation systems 150 that are provided in the left-hand and right-hand measurement optical systems 21 to start to image anterior segments of the left-hand and right-hand subject eyes E. The control unit 26 controls the display unit 30 of the tester controller 27 to cause the display screen 30a to display left-hand and right-hand anterior segment images (front images) E' based on image signals that have been output from the imaging elements 159 of the anterior segment observation systems 150.

Next, the tester performs an operation input to start alignment by using the input unit 30b of the tester controller 27. In step S2, the control unit 26 that has received input information (a control signal) that corresponds to this operation input controls the optotype projection systems 140 to display a fixation optotype (for example, a point light source optotype) in center positions of the optotype charts 143, and present the fixation optotype to the subject eyes E. In this state, the tester instructs that the subject fix the eyes to the fixation optotype.

In step S3 that follows, in a state where the subject has been allowed to fix the eyes to the fixation optotype, under the control of the control unit 26, the Z-alignment systems 110 perform alignment in the Z-direction on the measurement heads 16, and the XY-alignment systems 120 perform alignment in the X-direction and the Y-direction on the measurement heads 16.

In step S4 that follows, in order to calculate correction coefficients for detecting the line-of-sight direction of the subject eyes E, the control unit 26 controls the display unit 30 on the basis of an operation input performed by the tester by using the input unit 30b or automatically to cause the display screen 30a to display the operation screen 40, as illustrated in FIG. 4.

In step S5 that follows, the control unit 26 causes an optotype for the "front gaze" to be displayed in the first presentation positions in the center of the optotype charts 143 in accordance with an optotype selection operation performed by the tester. The tester instructs that the subject fix the eyes to the optotype. Next, in step S6, the control unit 26 detects bright-spot center-of-gravity coordinates and detects pupil center coordinates on the basis of anterior segment images E' of the "front gaze" that have been acquired by the imaging elements 159.

In step S7 that follows, the control unit 26 causes an optotype for the "left gaze" to be displayed, for example, in the second presentation positions on a left-hand side in the optotype charts 143 in accordance with an optotype selection operation performed by the tester. The tester instructs that the subject fix the eyes to the optotype. Next, in step S8, the control unit 26 detects bright-spot center-of-gravity coordinates and detects pupil center coordinates on the basis of anterior segment images E' of the "left gaze" that have been acquired by the imaging elements 159.

Note that in steps S6 and S8, the control unit 26 may automatically calculate the bright-spot center-of-gravity coordinates and the pupil center coordinates from the anterior segment images E' at an appropriate timing. Alternatively, the control unit 26 may calculate these coordinates at a timing at which the subject has operated the tester controller, this prevents calculation from being performed in a state where the subject has not fixed the eyes to the optotype, and the bright-spot center-of-gravity coordinates and the pupil center coordinates can be more appropriately calculated.

In step S9 that follows, the control unit 26 calculates correction coefficients on the basis of acquired feature points by using Formulae (3) and (4) described above. In step S10 that follows, the control unit 26 detects a line-of-sight direction at a time when the subject eyes E face an arbitrary direction (for example, a line-of-sight direction at the time of phoria testing or strabismus testing) on the basis of the anterior segment images E' of the subject eyes E. Specifically, the control unit 26 calculates prism amounts ($\Delta$) in the horizontal direction and the vertical direction as the line-of-sight direction of the subject eyes E, by using Formulae (1) and (2) described above. These prism amounts are determined to be data relating to the line-of-sight direction.

In step S11 that follows, the control unit 26 controls the display unit 30 to cause the display screen 30a to display the anterior segment images E' of the subject eyes E in the anterior segment image display regions 47 of the information display screen 40A, and display the data relating to the line-of-sight direction (the prism amounts in the horizontal direction and the vertical direction) in the line-of-sight direction display region 48, as illustrated in FIG. 7. The tester visually recognizes each of the images on this display screen 30a to be able to grasp whether the subject eyes E have phoria or strabismus, and a condition (a level) of an eye position, such as phoria or strabismus. Furthermore, the tester can check whether the subject eyes E are appropriately fixed to the optotype, malingering can be avoided, for example, and when phoria testing or strabismus testing is conducted by using the ophthalmic apparatus 100, as described below, the control unit 26 and the tester can effectively utilize the data relating to the line-of-sight direction.

Note that even for normal subject eyes E without strabismus, the center of the pupil and the center of gravity of a bright spot deviate from each other in the front gaze.

Furthermore, the ophthalmic apparatus 100 may perform detection of the line-of-sight direction and the display of the anterior segment images E' and the data relating to the line-of-sight direction only once at a predetermined timing. Furthermore, the ophthalmic apparatus 100 may always (repeatedly) perform these processes while the anterior segment images E' are acquired, and may present, to the tester or the like, the anterior segment images E' and the data relating to the line-of-sight direction that have been detected in real time.

In step S12 that follows, the control unit 26 controls the left-hand and right-hand measurement optical systems 21 to conduct objective testing, on the basis of an operation input performed by the tester to instruct objective testing by using the input unit 30b or automatically. Examples of the objective testing include corneal shape measurement (keratometry) performed by the keratometry system 130, ocular refractive power (refraction) measurement performed by the refraction measurement projection system 160 and the refraction measurement light-receiving system 170, and the like. By visually recognizing the anterior segment images E' and the line-of-sight direction (an amount of phoria or an amount of strabismus) that have been displayed on the display screen 30a in step S10, the tester can grasp a condition of an eye position of the subject eyes E, and can also grasp whether the subject appropriately fixes the eyes to an optotype, whether the head is steady, or the like. Therefore, in a case where the eyes are not appropriately fixed to the optotype, the tester can instruct that the subject fix the eyes to the optotype or can take measures to, for example, hold the head in order to not make the head unsteady, in advance, and objective measurement can be appropriately performed. This enables improvements in the efficiency of measurement or a reduction in a measurement error.

In step S13 that follows, subjective testing can be conducted on the subject eyes E. The tester performs a tap operation on the testing distance setting region 42 of the operation screen 40 to be able to change a testing distance, or performs a tap operation on the optotype icon 43 to be able to select optotypes to be presented to the subject eyes E. Furthermore, when monocular subjective testing is conducted, the tester performs a tap operation on one of the anterior segment images E' displayed in the anterior segment image display regions 45 of the operation screen 40 to be able to shied one of the subject eyes E. Furthermore, the control unit 26 may change a position of an optotype to be presented to the subject eyes E or may dispose a corrective lens in front of the subject eyes E on the basis of the line-of-sight direction (the amount of phoria or the amount of strabismus) that has been acquired in step S10, automatically or in response to an operation input performed by the tester. By doing this, the ophthalmic apparatus 100 enables subjective testing that corresponds to an eye position of the subject eyes E to be conducted.

Furthermore, the tester visually recognizes the anterior segment images E' and the line-of-sight direction (the amount of phoria or the amount of strabismus) that have been displayed on the display unit 30 to be able to check a line-of-sight direction at a time when the character arrangement optotypes are presented or check whether the subject has fixed the eyes to an optotype. Furthermore, malingering can be avoided, for example, and when phoria testing or the like is conducted, the tester or the control unit 26 can effectively utilize data relating to the line-of-sight direction. Furthermore, in testing using a perimeter, similarly, the tester or the control unit 26 can effectively utilize the data relating to the line-of-sight direction.

Then, on the basis of an optotype selection input performed by the tester by using the input unit 30b, the control unit 26 controls the optotype projection systems 140 to display an optotype in the optotype charts 143 and present the optotype to the subject eyes E, and to display the same optotype in the optotype display region 44. In this case, in order to orient optic axes of the subject eyes E to correspond to a testing distance, the control unit 26 may drive the left-hand and right-hand driving mechanisms 15 according to the testing distance to rotate the left-hand and right-hand measurement heads 16 in the X-direction.

The tester allows the subject to answer how an optotype looks in a state where the optotype is presented to the subject eyes E, and therefore the subjective testing is conducted. The tester performs a touch operation on the input unit 30b to appropriately change correction values for sphere power, cylinder power, the angle of an astigmatic axis, and the like in accordance with the presented optotype and whether the subject's answer is correct or not. The control unit 26 controls the measurement optical systems 21 on the basis of correction values after a change. As a result, correction values of the subject eyes E that are used by the measurement optical systems 21 have been changed, and the subject can undergo subjective testing by using the changed correction values.

If subjective testing has been repeated, prescription has been determined, and the tester has performed a termination operation, the program moves on to an end, and an operation performed by the ophthalmic apparatus 100 to acquire information relating to the subject eyes E (conduct testing) is terminated.

As described above, the ophthalmic apparatus 100 according to the first embodiment includes the imaging element 159 (the image acquisition unit) that acquires an anterior segment image E' of a subject eye E, the optotype projection system 140 that presents an optotype to the subject eye E in at least two different presentation positions, and the control unit 26 serving as a line-of-sight direction detection unit that extracts a feature point from each of the anterior segment images E' that have been acquired by the imaging element 159 when the optotype has been presented in the respective presentation positions, detects positional information on the anterior segment image E' of each of the extracted feature points, and detects a line-of-sight direction of the subject eye E on the basis of the positional information.

By employing this configuration, the ophthalmic apparatus 100 according to the first embodiment can detect the line-of-sight direction of the subject eye E more simply and with higher precision on the basis of the anterior segment images E'. Therefore, the ophthalmic apparatus 100 does not need to forcibly switch binocular vision and monocular vision as is conventional, measure adjustment of subject eyes before and after switching by using invisible light, and measure a change in a line-of-sight direction.

Accordingly, a tester or the ophthalmic apparatus 100 can appropriately grasp states of the subject eyes on the basis of the detected line-of-sight direction. As a result, the tester or the like can check whether the subject eyes E are appropriately fixed to an optotype, malingering can be avoided, for example, and when phoria testing, strabismus testing, or the like is conducted, the ophthalmic apparatus 100 and the tester can effectively utilize data relating to a line-of-sight direction.

Furthermore, in the ophthalmic apparatus 100 according to the first embodiment, the control unit 26 detects information relating to an eye position of the subject eyes E and more specifically, an amount of phoria or an amount of strabismus on the basis of the detected line-of-sight direction. This enables the control unit 26 or the tester to appropriately grasp a condition of the eye position of the subject eyes E, such as phoria or strabismus.

Furthermore, in the ophthalmic apparatus 100 according to the first embodiment, the control unit 26 acquires a first feature point from a bright spot image Br of the anterior segment image E', acquires a second feature point from a pupil image, and detects the line-of-sight direction on the basis of the first feature point and the second feature point. In this case, the control unit 26 detects bright-spot center-of-gravity coordinates (X, Y) as the first feature point, and detects pupil center coordinates (X', Y') as the second feature point. Then, the control unit 26 calculates a prism amount in a horizontal direction and a prism amount in a vertical direction of the line-of-sight direction of the subject eyes E relative to a predetermined reference direction according to Formulae (1) and (2) described above. By employing this configuration, the ophthalmic apparatus 100 can grasp the condition of the eye position of the subject eyes E, that is, a condition of phoria or strabismus in more detail and quantitatively, by using the amount of phoria or the amount of strabismus.

Furthermore, in the ophthalmic apparatus 100 according to the first embodiment, the imaging element 159 and the optotype projection system 140 are provided in one-to-one correspondence to correspond to left-hand and right-hand subject eyes E. By employing this configuration, eye information and a line-of-sight direction of the subject eye E can be detected in binocular vision, or the eye information and the line-of-sight direction of the subject eye E can be detected in monocular vision.

An ophthalmic apparatus according to the present disclosure has been described above on the basis of the embodiment. However, a specific configuration is not limited to this embodiment, and a design change, additions, or the like can be made without departing from the invention specified in each of the claims.

For example, the ophthalmic apparatus 100 according to the first embodiment described above presents an optotype while changing a display position on the optotype chart 143 to change the line-of-sight direction, but this is not restrictive. For example, the ophthalmic apparatus 100 can be configured to present an optotype in a plurality of presentation positions different from each other to the subject eye E, by disposing a corrective lens such as a prism lens having a known prism amount in front of the subject eye E or by driving the driving mechanism 15 to change an orientation of the measurement head 16.

Furthermore, the ophthalmic apparatus 100 according to the first embodiment described above uses character arrangement optotypes to detect the line-of-sight direction, but the character arrangement optotypes are not restrictive, and any type of optotype may be used if the line-of-sight direction of the subject eye E can be changed. For example, the optotype may be a fixed optotype, as illustrated as a landscape chart in FIG. 9. In this landscape chart illustrated in FIG. 9, a prism amount of an image of a house in the center has been set to 0Δ, and prism amounts of left-hand and right-hand ends of the horizon have been set to 8Δ. Therefore, a tester instructs that a subject fix the eyes to the house in the center, and therefore anterior segment images E' in the "front gaze" can be acquired. The tester instructs that the subject fix the eyes to the right-hand end (or the left-hand end) of the horizon, and therefore anterior segment images E' of the "right gaze" (or the "left gaze") at a position of 8Δ can be acquired.

Furthermore, in the ophthalmic apparatus 100 according to the first embodiment, in detecting the line-of-sight direction and causing the display unit 30 to display the anterior segment image E', the control unit 26 may cause the display unit 30 to conduct a display in a state where an image (a mapping image) quantitively indicating the line-of-sight direction is superimposed onto the anterior segment image E'. The left gaze 8Δ of FIG. 10 is a diagram illustrating a state where a prism circle image 50 is superimposed as the mapping image onto an anterior segment image E' of the left gaze at a prism amount of 8Δ. Each circle is drawn at intervals of 2Δ. Note that a center of a cross scale indicated in the anterior segment image E' of the left gaze 8Δ of FIG. 10, that is, a center C of a prism circle, is a position of a pupil center Pc in the front gaze. Note that a distance between the pupil center Pc and a corneal apex T (see FIGS. 11A and 11B) in the side gaze is very small, and therefore this center C can be regarded as a position of the corneal apex T. In the left gaze 8Δ of FIG. 10, the pupil center Pc is located in a fourth circle (8Δ) from the center C. Accordingly, the tester can more clearly and more quantitively grasp a condition of an eye position of the subject eyes E in real time on the basis of the position of the pupil center Pc on the prism circle image 50. In this case, the control unit 26 may control the display unit 30 to superimpose an image of an optotype (an optotype image 51) presented to the subject eye E, instead of the prism circle image 50. This optotype image 51 enables the tester to grasp in real time which place of the optotype the subject eye E is fixed to. An example of the optotype image 51 is illustrated in a lower portion of a page of FIG. 10, but the optotype image 51 is not limited to this example. Note that the anterior segment image E' is displayed on the display screen 30a in such a way that the subject eye E is visually recognized from a direction that faces the subject. Therefore, the optotype image 51 is displayed in a laterally inverted state, and this enables a line-of-sight direction and a prism amount of the subject eye E to be more appropriately grasped on the basis of a presentation position of the optotype. Furthermore, when detecting the line-of-sight direction by using the character arrangement optotypes illustrated in FIG. 4 or the like, similarly, the control unit 26 can superimpose a mapping image such as a prism circle (polar coordinates) image onto the anterior segment image E', or can superimpose an image of the character arrangement optotypes onto the anterior segment image E' in such a way that each optotype and a prism amount are correspondingly disposed. Note that a display mode of the prism circle is not limited to the example of FIG. 10, and for example, the prism circle may be enlarged and displayed to extend over the entire display region in order to make the tester easily view the prism circle. The prism circle is not limited to a circle of rectangular coordinates, and may have a rectangular shape (□). In displaying the optotype image 51, coordinate transformation is performed on the optotype image 51 in order to achieve correspondence in prism power.

Furthermore, in the first embodiment, the control unit 26 detects the line-of-sight direction of the subject eye E by using Formulae (1) and (2) described above, but this technique is not restrictive. For example, another technique for detecting the line-of-sight direction is described below with reference to FIGS. 11A and 11B.

The control unit 26 obtains, as the line-of-sight direction, an amount of displacement (a prism amount [Δ] and displacement $d_1$ described later) of a position of the bright spot image Br relative to a position of the pupil center Pc on the basis of bright-spot center-of-gravity coordinates and pupil center coordinates that serve as feature points acquired from the anterior segment image E'. This amount of displacement is regarded as data relating to the line-of-sight direction.

FIG. 11A illustrates a position of a bright spot Q in a subject eye E without strabismus, and FIG. 11B illustrates a position of a bright spot Q of a subject eye E having strabismus. The bright spot Q is formed in a position (r/2) of half of the radius of curvature r of the cornea. An image of the bright spot Q appears as the bright spot image Br on the anterior segment image E'. Furthermore, in FIGS. 11A and 11B, O is an eyeball rotation point, R is a center of corneal curvature, T is a corneal apex, and PX is an axis that passes through the pupil and the eyeball rotation point. Furthermore, Ep is a pupil image, and Ir is an iris image.

Incidentally, the radius of curvature r of the cornea (that is, a distance from the center of corneal curvature R to the corneal apex T), a distance d between a position of the corneal apex T and a position of the bright spot image Br, and the angle θ illustrated in FIG. 11B that is formed by the axis PX that passes through the pupil and the eyeball rotation point, and a parallel light flux are expressed by a relational expression, as described below in Formula (5).

$$\sin\theta = d/r \qquad (5)$$

The distance d and the radius of curvature r are substituted in Formula (5) described above, and therefore the angle θ can be calculated. As the radius of curvature r of the cornea, a value acquired in keratometry can be used. Furthermore, as the radius of curvature r of the cornea, a mean value (7.7 mm) may be used as an initial value.

In this variation, the control unit 26 obtains the angle θ according to Formula (6) described below, by using an amount of displacement (a distance $d_0$) of a position of the bright spot image Br relative to a position of the pupil center Pc and a distance $r_0$ from the center of corneal curvature R to the pupil center Pc. As a result, the control unit 26 can more efficiently calculate the angle θ or the like on the basis of the anterior segment image E'.

$$\sin\theta = d_0/r_0 \qquad (6)$$

The angle θ can be calculated by substituting the distance $d_0$ obtained in advance in Formula (6) described above. As the distance $r_0$, a mean value can be used, for example. Specifically, the distance $r_0$ can be obtained by subtracting the distance $r_1$ between the corneal apex T and the pupil center Pc from the radius of curvature r of the cornea. In a case where it is assumed that mean value of r = 7.7 mm, and mean value of $r_1$ = 3.6 mm (where a mean value in a case where the pupil center Pc is located on a front face of the lens), distance $r_0$ = (7.7 - 3.6) mm = 4.1 mm is established.

Note that a position of each of the pupil center Pc and the bright spot image Br is easily affected by refraction of the cornea, and the distance $r_0$ has individual differences. Therefore, the distance $d_0$ and the distance $r_0$ may be collected for an axis PX that passes through the pupil and an eyeball rotation point of a subject eye E without strabismus, as illustrated in FIG. 11A, and axes PX that pass through the pupil and an eyeball rotation point of a subject eye E that faces various directions, and the distance $r_0$ may be optimized on the basis of simultaneous equations of them. Alternatively, the distance $r_0$ may be optimized on the basis of a measured value of a radius of curvature r of the cornea that has been acquired in keratometry.

Furthermore, the angle θ can also be calculated according to Formula (7) described below instead of a calculation procedure using Formula (5) or (6) described above. In Formula (7) described below, L indicates a distance from the corneal apex T to the eyeball rotation point O, and D indicates a distance between a position of the corneal apex T and a position of the eyeball rotation point O. Note that the distance L from the corneal apex T to the eyeball rotation point O may be a predetermined value (for example, 13 mm, which is an average value). Alternatively, in a case where an actual distance is known by performing measurement using other equipment, the ophthalmic apparatus 100 may use this value as an input of the distance L. In this case, similarly, the control unit 26 may perform calculation by using a distance from the pupil center Pc to the eyeball rotation point O instead of the distance L, and using a distance between the pupil center Pc and a position of the eyeball rotation point O in the anterior segment image E' instead of the distance D.

$$\sin\theta = D/L \qquad (7)$$

As yet another technique for calculating the angle θ, for example, displacement $d_1$ (see FIG. 11B) of the bright spot image Br can be used. Only a subject eye E in which displacement has been detected is fixed to a fixation optotype, each numerical value is obtained in the state of FIG. 11A, and the displacement $d_1$ can be expressed as an amount of displacement of the bright spot image Br from the eyeball rotation point O. This displacement $d_1$ corresponds to the amount of strabismus (the prism amount [Δ]).

It is assumed that a distance from the corneal apex T to the eyeball rotation point O is L, and a radius or curvature of the cornea is r. In this case, the displacement $d_1$ illustrated in FIG. 11B of the bright spot image Br is expressed as described below in Formula (8). In this case, similarly, the distance L from the corneal apex T to the eyeball rotation point O may be a predetermined value (for example, 13 mm, which is an average value). Alternatively, in a case where an actual distance is known by performing measurement using other equipment, this value may be able to be input. As the radius of curvature r of the cornea, a value acquired in keratometry or a mean value (7.7 mm) can be used.

$$d_1 = (L - r)\cdot\sin\theta \qquad (8)$$

Furthermore, in the ophthalmic apparatus 100 according to the first embodiment, the control unit 26 can be configured to detect anterior chamber depth of a subject eye E in addition to detection of the line-of-sight direction. The control unit 26 can calculate the anterior chamber depth, as described below, on the basis of the bright-spot center-of-gravity coordinates (X, Y) and the pupil center coordinates (X', Y') that serve as feature points with reference to FIG. 11B, for example.

A bright-spot center-of-gravity eccentricity (X1 - X0) and a distance $L_1$ from the center of corneal curvature R to the eyeball rotation point O is expressed by a relational expression, as described below in Formula (9). A pupil center eccentricity (X1' - X0') and a distance $L_2$ from the pupil center Pc to the eyeball rotation point O is expressed by a relational expression, as described below in Formula (10). Furthermore, the prism amount P and the angle θ are expressed by a relational expression, as described below in Formula (11).

$$\text{Bright-spot center-of-gravity eccentricity } (X1 - X0) = L_1 \sin\theta \qquad (9)$$

$$\text{Pupil center eccentricity } (X1' - X0') = L_2 \sin\theta \qquad (10)$$

$$\tan\theta = P/100 \qquad (11)$$

The control unit 26 calculates the distance $L_1$, the distance $L_2$, and P according to Formulae (9) to (11) described above, and calculates the anterior chamber depth of the subject eye E according to Formula (12) described below. In Formula (12) described below, r is a radius of curvature of the cornea (a distance from the center of corneal curvature R to the corneal apex T), L is a distance from the corneal apex T to the eyeball rotation point O, $L_1$ is a distance from the center of corneal curvature R to the eyeball rotation point O, and $L_2$ is a distance from the pupil center Pc to the eyeball rotation point O. A thickness of the cornea may be determined to a predetermined value (for example, 530 μm, which is an average value). Alternatively, in a case where an actual distance is known by performing measurement using other equipment, this value may be able to be input.

Center anterior chamber depth = $L - L_2$ - thickness of cornea = $r + L_1 - L_2$ - thickness of cornea (12)

If the control unit 26 has calculated the anterior chamber depth, as described above, the control unit 26 can control the display unit 30 to cause the display screen 30a to display the anterior chamber depth together with the line-of-sight direction. By visually recognizing this display screen 30a, the tester or the like can grasp a condition of an eye position such as phoria or strabismus of the subject eyes E on the basis of the line-of-sight direction, and can also grasp a risk of decease other than phoria or strabismus, for example a risk of glaucoma, of the subject eyes E on the basis of the anterior chamber depth.

Yet another technique for calculating the angle θ and yet another technique for superimposing the prism circle image 50 are described below. As described above, the distance between the pupil center Pc and the corneal apex T is very small. Therefore, the control unit 26 may calculate the corneal apex T by using the technique described below, or may superimpose the prism circle image 50 or the like on the anterior segment image E' by using the technique described below.

For example, in a case whether the subject has appropriately applied the forehead to the forehead applied portion 17, and has only moved a line of sight from the front gaze without moving the face, and has performed a side gaze, the eyeball rotation point O of the anterior segment image E' does not deviate from the image. Therefore, the control unit 26 may draw a cross scale having the eyeball rotation point O of the subject eye E as a center on the anterior segment image E', and may superimpose a prism circle image 50 having the corneal apex T as the center C onto the anterior segment image E'. In this case, the angle θ can be calculated according to Formula (13) described below on the basis of the distance $L_1$ from the center of corneal curvature R to the eyeball rotation point O and the displacement $d_1$ of the bright spot image Br that are illustrated in FIG. 11B.

$$\sin\theta = d_1/L_1 \qquad (13)$$

In contrast, even in a case where the subject has moved the line of sight from the front gaze while moving the face, and has performed the side gaze, the control unit 26 performs image analysis on the anterior segment image E' to detect outer and inner corners of the eye, and can recognize a movement of the subject eye. The control unit 26 performs image analysis on the basis of this movement state to extract an eyeball rotation point O and a corneal apex T at the time of the side gaze, superimposes a cross scale having the eyeball rotation point O as a center and a prism circle image 50 having the corneal apex T as a center on the anterior segment image E', and displays a position of the eyeball rotation point O at the time of the front gaze in the anterior segment image E', and therefore the control unit 26 can report to the tester that the subject's face has moved.

Furthermore, the control unit 26 can generate a prism circle image 50 indicating a relationship between the eyeball rotation point O and the bright spot image Br, and can superimpose the prism circle image 50 onto the anterior segment image E'. Alternatively, the control unit 26 can generate a prism circle image 50 indicating a relationship between the eyeball rotation point O and the pupil center Pc, and can superimpose the prism circle image 50 onto the anterior segment image E'. In these cases, similarly, when the subject's face has moved in the side gaze, it is preferable that the control unit 26 detect outer and inner corners of the eye, generate each prism circle image 50 on the basis of a state of a movement of the subject eye E, and superimpose each of the prism circle images 50 onto the anterior segment image E'.

Furthermore, the control unit 26 can also generate a prism circle image indicating a relationship between the bright spot image Br and the pupil center Pc, and can superimpose the prism circle image onto the anterior segment image E'. In this case, similarly, even if the subject's face has moved in the side gaze, the control unit 26 can appropriately extract the bright spot image Br and the pupil center Pc by performing image analysis, and can appropriately perform generation and superimposition of a prism circle image 50.

Furthermore, the control unit 26 can calculate a position of the corneal apex T on the basis of the angle θ, can generate a prism circle image indicating a relationship between the bright spot image Br and the calculated corneal apex T, and can superimpose the prism circle image onto the anterior segment image E'. The angle θ can be calculated according to a trigonometric function expression or the like by using the distance L, the distance $L_1$, the distance $L_2$, the radius of curvature r, the distance $r_0$, the distance $r_1$, the distance D, the distance d, the distance $d_0$, the displacement $d_1$ or the like, as described above.

Effects of superimposing the prism circle image 50 or the optotype image 51 onto the anterior segment image E', as described above are described below. Conventionally, in a case where optometry is conducted, a tester needs to check a posture or a line-of-sight direction of a subject, check a presented optotype, or check a result of testing by using a controller, and this results in troublesome tasks. In contrast, the ophthalmic apparatuses 100 according to the embodiment described above and the variations described above, the anterior segment image E', and the prism circle image 50 or the optotype image 51 that is superimposed onto the anterior segment image E' are displayed as the information display screen 40A on the display unit 30 of the tester controller 27 that is operated by the tester. Therefore, the tester only visually recognizes the display unit 30 of the tester controller 27, and therefore the tester can not only visually recognize the subject eye E, but can also clearly and appropriately grasp a state of phoria, such as an amount of phoria or a direction of phoria, of the subject eye E, a presented optotype, a position of the optotype that the subject eye E is visually recognizing, or the like. Therefore, the tester can efficiently and appropriately perform a task for optometry. Stated another way, the ophthalmic apparatuses 100 according to the first embodiment described above and the variations described above does not only enable the tester or the like to appropriately grasp the condition of an eye position, such as strabismus or phoria, of the subject eyes E, but also enables usability for the tester to be improved.

Furthermore, as a variation of the ophthalmic apparatus 100 according to the first embodiment, the control unit 26 may have a function as a track display control unit that displays, on the optotype chart 143, tracks of an optotype to be presented to the subject eye E. Specifically, the control unit 26 causes the optotype chart 143 to present, for example, an optotype in the center, and then present an optotype on a left-hand side. In this case, the control unit 26 causes the optotype chart 143 to dynamically display an image of tracks of a movement from a presentation position of the optotype in the center toward a presentation position of the optotype on the left-hand side serving as the next fixation target.

A light flux of a track image displayed on the optotype chart 143 is projected together with a light flux of the optotype onto the subject eye E. This track image attracts attention of the subject, and can reliably guide a fixation position of the subject eye E from the presentation position of the optotype in the center to the presentation position of the optotype on the left-hand side. As a result, the control unit 26 can detect a line-of-sight direction with higher precision, and can more appropriately avoid malingering or the like. Furthermore, the optotype is not limited to character arrangement optotypes or a landscape chart, and any type of optotype may be used if a line-of-sight direction of the subject eye E can be changed. It is effective to enlarge or reduce an optotype and display the optotype, and this reliably enables the subject eye E to be fixed to the optotype.

Furthermore, the ophthalmic apparatus 100 according to the first embodiment can acquire a pupil image and an iris image from the anterior segment image E', and can use pupil center coordinates and iris center coordinates and can further use a pattern of the iris in order to detect the line-of-sight direction. In this case, in the ophthalmic apparatus 100, it is desirable that the anterior segment observation system 150 be configured to be able to acquire a color image by irradiating the subject eye E with visible light, and an anterior segment image E' including a clearer iris image can be acquired, and can be effectively used to detect the line-of-sight direction.

Furthermore, in the ophthalmic apparatus 100 according to the first embodiment, the measurement optical system 21 may include a camera (what is called a stereoscopic camera) that captures an anterior segment image E' of the subject eye E from plural different directions, separately from the imaging element 159. Such a camera can acquire an image indicating a wider range of the subject eye E including an inner corner, an outer corner, an eyelid, or the like in addition to the anterior segment image E'. Note that the ophthalmic apparatus 100 can also acquire an image indicating a wider range by including a wide-angle camera instead of the stereoscopic camera, or by disposing a wide-angle lens in the anterior segment observation system 150 to cause the imaging element 159 to form an image. The control unit 26 can grasp a movement of the face on the basis of such an image indicating a wider range, by using, for example, a change in positions of inner and outer corners of the subject eye E, and can correct the line-of-sight direction on the basis of this movement of the face. Therefore, the control unit 26 can more appropriately acquire information relating to the subject eye E, such as the line-of-sight direction, even in a case where the subject's face is not fixed.

## Claims

**1.** An ophthalmic apparatus comprising:

an image acquisition unit (159) configured to acquire an anterior segment image (E') of a subject eye (E);
an optotype projection system (140) configured to present a fixation optotype to the subject eye (E) in at least two presentation positions different from each other;
a control unit (26) configured to change a presentation position of the optotype on an optotype chart (143) to change the line-of-sight direction of the subject eye (E);
a line-of-sight direction detection unit (26) configured to extract a first feature point and a second feature point from the anterior segment image (E') that has been acquired by the image acquisition unit (159) when the optotype has been presented in each of the at least two presentation positions, detect positional information on the anterior segment image (E') of the first feature point and the second feature point that have been extracted, and configured to detect a line-of-sight direction of the subject eye (E) on a basis of the positional information, and
an anterior segment illumination light source (151) configured to irradiate the anterior segment of the subject eye (E) with illumination light that is constituted by a parallel light flux,
wherein the line-of-sight direction detection unit (26) is configured to acquire the first feature point from corneal reflex based on a point image obtained by the parallel light flux that enters the subject eye (E) as an image in the subject eye (E), is configured to acquire the second feature point from a pupil image that has been detected from the anterior segment image (E'), and is configured to detect the line-of-sight direction on a basis of the first feature point and the second feature point,
wherein:

the line-of-sight direction detection unit (26) is configured to detect bright-spot center-of-gravity coordinates (X, Y) as the first feature point, is configured to detect pupil center coordinates (X', Y') as the second feature point, and is configured to calculate a prism amount in a horizontal direction and a prism amount in a vertical direction of the line-of-sight direction of the subject eye (E) relative to a predetermined reference direction according to formulae described below:

$$\text{Line-of-sight direction (horizontal) of subject eye } [\Delta] = a*(X' - X) + b;$$

and

$$\text{Line-of-sight direction (vertical) of subject eye } [\Delta] = a'*(Y' - Y) + b',$$

where a, b, a', and b' are correction coefficients and are calculated according to formulae,

$$a = P/((X1' - X1) - (X'0 - X0))$$

$$b = -P*(X0' - X0)/((X1' - X1) - (X'0 - X0)),$$

wherein X0 is an X-coordinate of the center of gravity of the bright spot in a line-of sight direction at a time when an optotype is presented in a first presentation position,
wherein X0' is an X-coordinate of a pupil center (Pc) in a line-of-sight direction at a time when an optotype is presented in the first presentation position,
wherein X1 is an X-coordinate of the center of gravity of a bright spot in a line-of-sight direction at a time when an optotype is presented in a second presentation position,
wherein X1' is an X-coordinate of the pupil center (Pc) in a line-of-sight direction at a time when an optotype is presented in the second presentation position,
wherein P is a prism amount of an optotype that is presented to the subject eye (E).

**2.** The ophthalmic apparatus according to claim 1, wherein the line-of-sight direction detection unit (26) is configured to detect a condition of an eye position of the subject eye (E) on a basis of the line-of-sight direction that has been detected.

**3.** The ophthalmic apparatus according to claim 1, further comprising:

a display unit (30) configured to display the anterior segment image (E'); and
a display control unit configured to cause the display unit (30) to conduct a display in a state where an image quantitively indicating the line-of-sight direction is superimposed onto the anterior segment image (E').

**4.** The ophthalmic apparatus according to any one of claims 1 to 3 comprising:

a measurement unit (16) that includes:

a measurement optical system (21) configured to acquire information relating to the subject eye (E);
the image acquisition unit (159) configured to acquire the anterior segment image (E') on an optical axis of the measurement optical system (21) of the subject eye (E); and
the optotype projection system (140);

a driving mechanism (15) configured to move the measurement unit in a vertical direction and a horizontal direction, and configured to rotate the measurement unit (16) by using, as a rotation axis, an axis that is parallel to the vertical direction and an axis that is parallel to the horizontal direction; and
a display unit (30) configured to display the anterior segment image (E').

**Patentansprüche**

**1.** Ophthalmische Vorrichtung, umfassend:

eine Bilderfassungseinheit (159), die konfiguriert ist, ein Bild des vorderen Segments (E') eines Patientenauges (E) zu erfassen;
ein Optotypen-Projektionssystem (140), das konfiguriert ist, dem Patientenauge (E) einen Fixationsoptotyp in mindestens zwei voneinander unterschiedlichen Darstellungspositionen darzustellen;
eine Steuereinheit (26), die konfiguriert ist, eine Darstellungsposition des Optotyps auf einer Optotyp- Tafel (143) zu ändern, um die Blickrichtung des Patientenauges (E) zu ändern;
eine Blickrichtungsdetektionseinheit (26), die konfiguriert ist, einen ersten Merkmalspunkt und einen zweiten Merkmalspunkt aus dem Bild des vorderen Segments (E') zu extrahieren, das von der Bilderfassungseinheit (159) erfasst wurde, wenn der Optotyp in jeder der mindestens zwei Darstellungspositionen dargestellt wurde, Positionsinformationen auf dem Bild des vorderen Segments (E') des ersten Merkmalspunkts und des zweiten Merkmalspunkts, die extrahiert wurden, zu detektieren und konfiguriert ist, eine Blickrichtung des Patientenauges (E) auf Basis der Positionsinformation zu detektieren, und
eine Beleuchtungslichtquelle (151) für das vordere Segment, die konfiguriert ist, das vordere Segment des Patientenauges (E) mit Beleuchtungslicht zu bestrahlen, das durch einen parallelen Lichtstrom gebildet wird,
wobei die Blickrichtungsdetektionseinheit (26) konfiguriert ist, den ersten Merkmalspunkt aus dem Kornealreflex auf Basis eines Punktbildes zu erfassen, das durch den parallelen Lichtstrom erhalten wird, der in das Patientenauge (E) als ein Bild in dem Patientenauge (E) eintritt, konfiguriert ist, den zweiten Merkmalspunkt aus einem Pupillenbild zu erfassen, das aus dem Bild des vorderen Segments (E') detektiert wurde, und konfiguriert ist, die Blickrichtung auf Basis des ersten Merkmalspunkts und des zweiten Merkmalspunkts zu detektieren,
wobei:

die Blickrichtungsdetektionseinheit (26) konfiguriert ist, Schwerpunktkoordinaten (X, Y) des hellen Punkts als den ersten Merkmalspunkt zu detektieren, konfiguriert ist, Pupillenmittelpunktkoordinaten (X', Y') als den zweiten Merkmalspunkt zu detektieren, und konfiguriert ist, einen Prismenwert in einer horizontalen Richtung und einen Prismenwert in einer vertikalen Richtung der Blickrichtung des Patientenauges (E) relativ zu einer vorbestimmten Referenzrichtung gemäß unten beschriebenen Formeln zu berechnen:

$$\text{Blickrichtung (horizontal) des Patientenauges } [\Delta] = a*(X' - X) + b;$$

und

$$\text{Blickrichtung (vertikal) des Patientenauges } [\Delta] = a'*(Y' - Y) + b',$$

wobei a, b, a' und b' Korrekturkoeffizienten sind und nach Formeln berechnet werden,

$$a = P/((X1' - X1) - (X'0 - X0))$$

$$b = -P*(X0' - X0)/((X1' - X1) - (X'0 - X0)),$$

wobei X0 eine X-Koordinate des Schwerpunkts des hellen Punkts in einer Blickrichtung zu einem Zeitpunkt ist, zu dem ein Optotyp in einer ersten Darstellungsposition dargestellt wird,
wobei X0' eine X-Koordinate eines Pupillenmittelpunkts (Pc) in einer Blickrichtung zu einem Zeitpunkt ist, zu dem ein Optotyp in der ersten Darstellungsposition dargestellt wird,
wobei X1 eine X-Koordinate des Schwerpunkts eines hellen Punkts in einer Blickrichtung zu einem Zeitpunkt ist, zu dem ein Optotyp in einer zweiten Darstellungsposition dargestellt wird,
wobei X1' eine X-Koordinate des Pupillenmittelpunkts (Pc) in einer Blickrichtung zu einem Zeitpunkt ist, zu dem ein Optotyp in der zweiten Darstellungsposition dargestellt wird,
wobei P ein Prismenwert eines Optotyps ist, der dem Patientenauge (E) dargestellt wird.

2. Ophthalmische Vorrichtung nach Anspruch 1, wobei die Blickrichtungsdetektionseinheit (26) konfiguriert ist, einen Zustand einer Augenposition des Patientenauges (E) auf Basis der Blickrichtung, die detektiert wurde, zu detektieren.

3. Ophthalmische Vorrichtung nach Anspruch 1, ferner umfassend:

eine Anzeigeeinheit (30), die konfiguriert ist, das Bild des vorderen Segments (E') anzuzeigen; und
eine Anzeigesteuereinheit, die konfiguriert ist, die Anzeigeeinheit (30) zu veranlassen, eine Anzeige in einem Zustand durchzuführen, in dem ein Bild, das die Blickrichtung quantitativ anzeigt, auf dem Bild des vorderen Segments (E') überlagert ist.

4. Ophthalmische Vorrichtung nach einem der Ansprüche 1 bis 3 umfassend:

eine Messeinheit (16) umfassend:

ein optisches Messsystem (21), das konfiguriert ist, Informationen in Bezug auf das Patientenauge (E) zu erfassen;
die Bilderfassungseinheit (159), die konfiguriert ist, das Bild des vorderen Segments (E') auf einer optischen Achse des optischen Messsystems (21) des Patientenauges (E) zu erfassen; und
das Optotypen-Projektionssystem (140);

einen Antriebsmechanismus (15), der konfiguriert ist, die Messeinheit in einer vertikalen Richtung und einer horizontalen Richtung zu bewegen, und der konfiguriert ist, die Messeinheit (16) zu drehen unter Verwendung einer Achse, die parallel zu der vertikalen Richtung ist, und einer Achse, die parallel zu der horizontalen Richtung ist, als eine Drehachse; und
eine Anzeigeeinheit (30), die konfiguriert ist, das Bild des vorderen Segments (E') anzuzeigen.

## Revendications

**1.** Appareil ophtalmique comprenant

une unité d'acquisition d'images (159) configurée pour acquérir une image du segment antérieur (E') d'un œil du sujet (E) ;
un système de projection d'optotypes (140) configuré pour présenter un optotype de fixation à l'œil du sujet (E) dans au moins deux positions de présentation différentes l'une de l'autre ;
une unité de commande (26) configurée pour modifier une position de présentation de l'optotype sur une mire d'optotype (143) afin de modifier la direction de la ligne de visée de l'œil du sujet (E);
une unité de détection de la direction de la ligne de visée (26) configurée pour extraire un premier point caractéristique et un deuxième point caractéristique de l'image du segment antérieur (E') qui a été acquise par l'unité d'acquisition d'images (159) lorsque l'optotype a été présenté dans chacune des au moins deux positions de présentation, détecter des informations de position sur l'image du segment antérieur (E') du premier point caractéristique et du deuxième point caractéristique qui ont été extraits, et configurée pour détecter une direction de la ligne de visée de l'œil du sujet (E) sur la base des informations de position, et
une source de lumière d'illumination du segment antérieur (151) configurée pour irradier le segment antérieur de l'œil du sujet (E) avec une lumière d'illumination qui est constituée par un flux de lumière parallèle,
dans lequel l'unité de détection de la direction de la ligne de visée (26) est configurée pour acquérir le premier point caractéristique à partir du reflet cornéen sur la base d'une image ponctuelle obtenue par le flux lumineux parallèle qui pénètre dans l'œil du sujet (E) sous forme d'image dans l'œil du sujet (E), est configurée pour acquérir le deuxième point caractéristique à partir d'une image pupillaire qui a été détectée à partir de l'image du segment antérieur (E'), et est configurée pour détecter la direction de la ligne de visée sur la base du premier point caractéristique et du deuxième point caractéristique,
dans lequel:

l'unité de détection de la direction de la ligne de visée (26) est configurée pour détecter les coordonnées du centre de gravité de la tache lumineuse (X, Y) en tant que premier point caractéristique, est configurée pour détecter les coordonnées du centre de la pupille (X', Y') en tant que deuxième point caractéristique, et est configurée pour calculer une quantité de prisme dans une direction horizontale et une quantité de prisme dans une direction verticale de la direction de la ligne de visée de l'œil du sujet (E) par rapport à une direction de référence prédéterminée conformément aux formules décrites ci-dessous :

$$\text{Direction de la ligne de visée (horizontale) de l'œil du sujet } [\Delta] = a*(X' - X) + b \text{ ;}$$

et

$$\text{Direction de la ligne de visée (verticale) de l'œil du sujet } [\Delta] = a'*(Y' - Y) + b',$$

où a, b, a', et b' sont des coefficients de correction et sont calculés selon des formules,

$$a = P/((X1' - X1) - (X'0 - X0))$$

$$b = -P*(X0' - X0)/((X1' - X1) - (X'0 - X0)),$$

dans lequel X0 est une coordonnée X du centre de gravité de la tache lumineuse dans une direction de la ligne de visée à un moment où un optotype est présenté dans une première position de présentation,
dans lequel X0' est une coordonnée X du centre de la pupille (Pc) dans une direction de la ligne de visée à un moment où un optotype est présenté dans la première position de présentation,
dans lequel X1 est une coordonnée X du centre de gravité de la tache lumineuse dans une direction de la ligne de visée à un moment où un optotype est présenté dans une deuxième position de présentation,
dans lequel X1' est une coordonnée X du centre de la pupille (Pc) dans une direction de ligne de visée à un moment où un optotype est présenté dans la deuxième position de présentation,
dans lequel P est la quantité de prisme d'un optotype présenté à l'œil du sujet (E).

**2.** Appareil ophtalmique selon la revendication 1, dans lequel l'unité de détection de la direction de la ligne de visée (26) est configurée pour détecter une condition de position de l'œil du sujet (E) sur la base de la direction de la ligne de visée qui a été détectée.

**3.** Appareil ophtalmique selon la revendication 1, comprenant en outre:

une unité d'affichage (30) configurée pour afficher l'image du segment antérieur (E'); et
une unité de commande d'affichage configurée pour amener l'unité d'affichage (30) à effectuer un affichage dans un état où une image indiquant quantitativement la direction de la ligne de visée est superposée à l'image du segment antérieur (E').

**4.** Appareil ophtalmique selon l'une quelconque des revendications 1 à 3, comprenant:

une unité de mesure (16) qui comprend:

un système optique de mesure (21) configuré pour acquérir des informations relatives à l'œil du sujet (E);
l'unité d'acquisition d'images (159) configurée pour acquérir l'image du segment antérieur (E') sur un axe optique du système optique de mesure (21) de l'œil du sujet (E); et
le système de projection de l'optotype (140) ;

un mécanisme d'entraînement (15) configuré pour déplacer l'unité de mesure dans une direction verticale et une direction horizontale, et configuré pour faire tourner l'unité de mesure (16) en utilisant, comme axe de rotation, un axe parallèle à la direction verticale et un axe parallèle à la direction horizontale ; et
une unité d'affichage (30) configurée pour afficher l'image du segment antérieur (E').

# FIG.1

100
14
10
15(15R)
17
15(15L)
21(21R)
21(21L)
Y
X
Z
16(16R)
18(18R)
30a,30b
30
13
18(18L)
16(16L)
12
27
26
11

FIG.2

21(21R)
113
112
140
147
146
145
144
143
142
141
A
150
130
151
168
Ef
E
152
153
155
156
157
158
159
26
30
30a
A
154
A,B
131
121
120
162
160
167
151
E´
O
B
163
A
161
166
171
110
173
174
170
175
172
111
165
164
A

FIG.3A

165
164

FIG.3B

163
163b
163a
165

FIG.4

30a,30b
30
27
SUBJECT INFORMATION
OBJECTIVE INFORMATION
SUBJECTIVE INFORMATION
OUTPUT
YYYY/MM/DD
hh:mm
RIGHT EYE
BOTH EYES
LEFT EYE
R
(-)
SPHERE
G
(+)
OBJECTIVE DATA
RIGTH
SUBJECTIVE DATA : DISTANT VIEW
LEFT
+1.25
SPHERE
+1.00
-1.75
ASTIGMATISM
-2.00
76
AXIS (5)
84
0.00
ADD
0.00
0.00
HORIZONTALITY
0.00
0.00
VERTICALITY
0.00
TEST DISTANCE
5.0
m
42
40
41
45
PD
63.5
EMERGENCY SHUTDOWN
ADJUST SPHERE POWER TILL SPHERICAL TARGET VISUAL ACUITY CHART (TYPICALLY ABOUT 0.4-0.5) IS SEEN
E´
44
43
SCA
VA
Bino
Near
Blank
0.2
0.3
0.4
P:0.5
BLOCK / SHIELDING
CLOUDY FOG
REFERENCE DATA
PRESCRIPTION : RECORD
DISTANT VIEW /CLOSE VIEW
TEST LIST
DATA ENTRY
DRIVING RESET
46

FIG.5

30a,30b
30
27
SUBJECT INFORMATION
OBJECTIVE INFORMATION
SUBJECTIVE INFORMATION
OUTPUT
YYYY/MM/DD
hh:mm
RIGHT EYE
BOTH EYES
LEFT EYE
R
SPHERE
G
(-)
(+)
OBJECTIVE DATA
RIGTH
SUBJECTIVE DATA : DISTANT VIEW
LEFT
TEST DISTANCE
5.0
m
+1.25
SPHERE
+1.00
-1.75
ASTIGMATISM
-2.00
76
AXIS(5)
84
0.00
ADD
0.00
HORIZONTALITY
VERTICALITY
PD
63.5
EMERGENCY SHUTDOWN
ADJUST SPHERE POWER TILL SPHERICAL TARGET VISUAL ACUITY CHART (TYPICALLY ABOUT 0.4-0.5) IS SEEN
42
40
41
45
45
E´
44
43
SCA
VA
Bino
Near
Blank
0.2
0.3
0.4
P:0.5
BLOCK / SHIELDING
CLOUDY FOG
REFERENCE DATA
PRESCRIPTION : RECORD
DISTANT VIEW /CLOSE VIEW
TEST LIST
DATA ENTRY
DRIVING RESET
46

# FIG.6

FRONT GAZE
Pc (PUPIL CENTER)
PUPIL CENTER COORDINATE(X0', Y0')
Br (BRIGHT SPOT IMAGE)
BRIGHT SPOT CENTER OF
GRAVITY COORDINATE(X0, Y0)
E´

P[Δ] OF LEFT GAZE
Pc (PUPIL CENTER)
PUPIL CENTER COORDINATE(X1', Y1')
E´
Br (BRIGHT SPOT IMAGE)
BRIGHT SPOT CENTER OF
GRAVITY COORDINATE(X1, Y1)

FIG.7

SUBJECT INFORMATION
OBJECTIVE INFORMATION
SUBJECTIVE INFORMATION
OUTPUT
YYYY/MM/DD
hh:mm
RIGTH
LEFT
PD
63.5
PD
63.5
PHORIA・AMOUNT OF STRABISMUS (HORIZONTALITY) : ○○[Δ]
PHORIA・AMOUNT OF STRABISMUS (VERTICALITY) : ○○[Δ]
P:0.5
BLOCK / CLOUDY FOG
SHIELDING
REFERENCE DATA
PRESCRIPTION : RECORD
DISTANT VIEW /CLOSE VIEW
TEST LIST
DATA ENTRY
DRIVING RESET

FIG.8

START
IMAGE ANTERIOR SEGMENT AND DISPLAY ANTERIOR SEGMENT IMAGE
S1
DISPLAY FIXATION OPTOTYPE
S2
ALIGNMENT
S3
DISPLAY OPERATION SCREEN
S4
DISPLAY OPTOTYPE FOR FRONT GAZE
S5
EXTRACT FEATURE POINT OF FRONT GAZE FROM ANTERIOR EYE IMAGE
S6
DISPLAY OPTOTYPE FOR LEFT GAZE
S7
EXTRACT FEATURE POINT OF LEFT GAZE FROM ANTERIOR EYE IMAGE
S8
CALCULATE CORRECTION COEFFICIENT
S9
DETECT ARBITRARY LINE OF SIGHT DIRECTION
S10
DISPLAY ANTERIOR EYE IMAGE IN WHICH DATA OF LINE OF SIGHT DIRECTION IS SUPERIMPOSED
S11
OBJECTIVE TEST
S12
DISPLAY OPTOTYPE OF SUBJECTIVE TEST
S13
END

FIG.9

FIG.10

FRONT GAZE
Pc
E´
Br
LEFT GAZE 8△
C
50(51)
Br
Pc
E´
EXAMPLE OF
OPTOTYPE IMAGE 51

FIG.11A
FIG.11B
PX
O
R
Q
L
Pc
T
r
$r_0$
$r_1$
$L_1$
$L_2$
D
θ
$d_0$
d
$d_1$
Ir
Ep
Pc,Br
Br

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5011144 B
- JP 2016158721 A
- JP 2021146184 A